(19) Europäisches Patentamt
European Patent Office
Office européen des brevets

(11) **EP 2 331 678 B1**

(12) **EUROPEAN PATENT SPECIFICATION**

(45) Date of publication and mention of the grant of the patent:
**30.09.2015 Bulletin 2015/40**

(21) Application number: **09763701.1**

(22) Date of filing: **12.06.2009**

(51) Int Cl.:
***C12N 5/10*** *(2006.01)* ***C12N 5/07*** *(2010.01)*

(86) International application number:
**PCT/US2009/047178**

(87) International publication number:
**WO 2009/152413 (17.12.2009 Gazette 2009/51)**

(54) **METHODS FOR OBTAINING HIGH VIABLE CELL DENSITY IN MAMMALIAN CELL CULTURE**

VERFAHREN ZUR ERZIELUNG EINER HOHEN DICHTE LEBENSFÄHIGER ZELLEN BEI DER SÄUGERZELLKULTUR

PROCÉDÉS DESTINÉS À L'OBTENTION D'UNE HAUTE DENSITÉ DE CELLULES VIABLES DANS DES CULTURES DE CELLULES DE MAMMIFÈRES

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **13.06.2008 US 61233 P**

(43) Date of publication of application:
**15.06.2011 Bulletin 2011/24**

(73) Proprietor: **Janssen Biotech, Inc.**
**Horsham, PA 19044 (US)**

(72) Inventors:
- **DORAI, Haimanti**
**Radnor**
**PA 19087 (US)**
- **KYUNG, Yun, Seung**
**Radnor**
**PA 19087 (US)**

(74) Representative: **Carpmaels & Ransford LLP**
**One Southampton Row**
**London WC1B 5HA (GB)**

(56) References cited:
**US-A- 5 672 502** **US-A1- 2005 089 993**
**US-A1- 2007 092 947** **US-A1- 2008 015 164**

- **BRUNO FIGUEROA ET AL: "Enhanced cell culture performance using inducible anti-apoptotic genes E1B-19K and Aven in the production of a monoclonal antibody with Chinese hamster ovary cells", BIOTECHNOLOGY AND BIOENGINEERING, vol. 97, no. 4, 1 July 2007 (2007-07-01), pages 877-892, XP055055041, ISSN: 0006-3592, DOI: 10.1002/bit.21222**
- **DE LA CRUZ EDMONDS M CELINA ET AL: "Development of transfection and high-producer screening protocols for the CHOK1SV cell system", MOLECULAR BIOTECHNOLOGY, HUMANA PRESS, INC, US, vol. 34, no. 2, 1 October 2006 (2006-10-01), pages 179-190, XP002508715, ISSN: 1073-6085, DOI: 10.1385/MB:34:2:179**
- **PEREZ D ET AL: "E1B 19K INHIBITS FAS-MEDIATED APOPTOSIS THROUGH FADD-DEPENDENT SEQUESTRATION OF FLICE", THE JOURNAL OF CELL BIOLOGY : JCB, THE ROCKEFELLER UNIVERSITY PRESS, vol. 141, no. 5, 1 June 1998 (1998-06-01), pages 1255-1266, XP002929049, ISSN: 0021-9525, DOI: 10.1083/JCB.141.5.1255**
- **HAIMANTI DORAI ET AL: "Expression of anti-apoptosis genes alters lactate metabolism of Chinese Hamster Ovary cells in culture", BIOTECHNOLOGY AND BIOENGINEERING, vol. 103, no. 3, 15 June 2009 (2009-06-15) , pages 592-608, XP055028895, ISSN: 0006-3592, DOI: 10.1002/bit.22269**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 331 678 B1

- SAUERWALD ET AL.: 'Combining caspase and mitochondrial dysfunction inhibitors of apoptosis to limit cell death in mammalian cell cultures.' BIOTECHNOLOGY AND BIOENGINEERING vol. 94, 2006, pages 362 - 372, XP008097424
- FIGUEROA ET AL.: 'Comparison of Bcl-2 to a Bcl-2 deletion mutant for mammalian cells exposed to culture insults.' BIOTECHNOLOGY AND BIOENGINEERING vol. 73, 2001, pages 211 - 222, XP008064159
- NIVICHANYONG ET AL.: 'Anti-apoptotic genes Aven and E1B-19K enhance performance of BHK cells engineered to express recombinant factor VIII in batch and low perfusion cell culture.' BIOTECHNOLOGY AND BIOENGINEERING vol. 98, 2007, pages 825 - 841, XP008097425
- CORY ET AL.: 'The Bcl-2 family: roles in cell survival and oncogenesis.' ONCOGENE vol. 22, 2003, pages 8590 - 8607, XP008143887
- YANG ET AL.: 'Prevention of apoptosis by Bcl-2: release of cytochrome c from mitochondria blocked.' SCIENCE vol. 275, 1997, pages 1129 - 1132, XP008143888

SEQUENCE LISTING

<110> DORAI, HAIMANTI KYUNG, YUN SEUNG

<120> Methods for Obtaining High Viable Cell Density in Mammalian Cell Culture

<130> CEN5222USNP

<140> TO BE ASSIGNED
<141> 2009-06-12

<150> 61/061233
<151> 2008-06-13

<160> 12

<170> FastSEQ for Windows Version 4.0

<210> 1
<211> 660
<212> DNA
<213> Homo sapiens

<400> 1

```
atgtcgtccc acctagtcga gccgccgccg cccctgcaca acaacaacaa caactgcgag 60
gaaaatgagc agtctctgcc cccgccggcc ggcctcaaca gttcctgggt ggagctaccc 120
atgaacagca gcaatggcaa tgataatggc aatgggaaaa atggggggct ggaacacgta 180
ccatcctcat cctccatcca caatggagac atggagaaga ttcttttgga tgcacaacat 240
gaatcaggac agagtagttc cagaggcagt tctcactgtg acagcccttc gccacaagaa 300
gatgggcaga tcatgtttga tgtggaaatg cacaccagca gggaccatag ctctcagtca 360
gaagaagaag ttgtagaagg agagaaggaa gtcgaggctt tgaagaaaag tgcggactgg 420
gtatcagact ggtccagtag acccgaaaac attccaccca aggagttcca cttcagacac 480
cctaaacgtt ctgtgtcttt aagcatgagg aaaagtggag ccatgaagaa aggggggtatt 540
ttctccgcag aatttctgaa ggtgttcatt ccatctctct tcctttctca tgttttggct 600
ttggggctag gcatctatat tggaaagcga ctgagcacac cctctgccag cacctactga 660
```

<210> 2
<211> 219
<212> PRT
<213> Homo sapiens

<400> 2

```
Met Ser Ser His Leu Val Glu Pro Pro Pro Pro Leu His Asn Asn Asn
 1               5                  10                  15
Asn Asn Cys Glu Glu Asn Glu Gln Ser Leu Pro Pro Pro Ala Gly Leu
            20                  25                  30
Asn Ser Ser Trp Val Glu Leu Pro Met Asn Ser Ser Asn Gly Asn Asp
        35                  40                  45
Asn Gly Asn Gly Lys Asn Gly Gly Leu Glu His Val Pro Ser Ser Ser
    50                  55                  60
Ser Ile His Asn Gly Asp Met Glu Lys Ile Leu Leu Asp Ala Gln His
65                  70                  75                  80
Glu Ser Gly Gln Ser Ser Ser Arg Gly Ser Ser His Cys Asp Ser Pro
                85                  90                  95
Ser Pro Gln Glu Asp Gly Gln Ile Met Phe Asp Val Glu Met His Thr
            100                 105                 110

Ser Arg Asp His Ser Ser Gln Ser Glu Glu Glu Val Val Glu Gly Glu
        115                 120                 125
Lys Glu Val Glu Ala Leu Lys Lys Ser Ala Asp Trp Val Ser Asp Trp
    130                 135                 140
Ser Ser Arg Pro Glu Asn Ile Pro Pro Lys Glu Phe His Phe Arg His
145                 150                 155                 160
Pro Lys Arg Ser Val Ser Leu Ser Met Arg Lys Ser Gly Ala Met Lys
                165                 170                 175
Lys Gly Gly Ile Phe Ser Ala Glu Phe Leu Lys Val Phe Ile Pro Ser
            180                 185                 190
Leu Phe Leu Ser His Val Leu Ala Leu Gly Leu Gly Ile Tyr Ile Gly
        195                 200                 205
Lys Arg Leu Ser Thr Pro Ser Ala Ser Thr Tyr
    210                 215
```

<210> 3
<211> 1089
<212> DNA
<213> Homo sapiens

<400> 3

```
atgcaggcgg agcgaggagc tcggggaggc cgtgggcggc ggccaggccg cggccggcct 60
ggcggagatc gccacagcga gcggcccgga gccgcagcgg cggtagccag aggcggcggc 120
ggaggcggcg gcggggacgg aggcggacgc cggggccgtg gccgtggccg gggcttccgc 180
ggcgctcgcg gaggccgagg aggaggaggc gcccccgcgag gcagccgccg ggagccggga 240
ggctggggcg caggggccag cgcgccggtt gaagatgaca gcgatgcaga gacctatgga 300
gaagagaatg atgaacaggg aaattattct aaaagaaaga ttgtctctaa ctgggatcga 360
tatcaagata ttgaaaaaga ggtcaataat gaaagtggag agtcacagag gggaacagat 420
ttcagtgtcc tccttagctc tgcaggggac tcattctcac agttccggtt tgctgaggag 480
aaagaatggg atagtgaagc ttcttgtcca aaacagaatt cagcatttta tgtggatagt 540
gagttattgg ttcgagccct tcaagagctg cctctctgcc tccgactcaa cgttgctgcc 600
gaactggtcc agggtacagt tcctttagag gttcctcagg tgaaaccaaa gagaactgat 660
gatggcaagg gattagggat gcagttaaag gggcccttgg ggcctggagg aagggggccc 720
atctttgagc tgaaatctgt ggctgctggc tgccctgtgt tgctgggcaa agacaaccca 780
agcccgggtc cttcaaggga ttctcagaaa cccacttccc cactgcagtc agcaggagac 840
catttggaag aagaactaga tctgttgctt aatttagatg cacctataaa agagggagat 900
aacatcttac cagatcagac gtctcaggac ctgaaatcca aggaagatgg ggaggtggtc 960
caagaggaag aagtttgtgc aaaaccatct gtgactgaag aaaaaaacat ggaacctgag 1020
caaccaagta cctccaaaaa tgttaccgag gaagagctgg aagactggtt ggacagcatg 1080
atttcctaa                                                         1089
```

<210> 4
<211> 362
<212> PRT
<213> Homo sapiens

<400> 4

```
Met Gln Ala Glu Arg Gly Ala Arg Gly Gly Arg Gly Arg Arg Pro Gly
 1               5                  10                  15
Arg Gly Arg Pro Gly Gly Asp Arg His Ser Glu Arg Pro Gly Ala Ala
            20                  25                  30
Ala Ala Val Ala Arg Gly Gly Gly Gly Gly Gly Gly Gly Asp Gly Gly
        35                  40                  45
Gly Arg Arg Gly Arg Gly Arg Gly Arg Gly Phe Arg Gly Ala Arg Gly
    50                  55                  60
Gly Arg Gly Gly Gly Gly Ala Pro Arg Gly Ser Arg Arg Glu Pro Gly
65                  70                  75                  80
Gly Trp Gly Ala Gly Ala Ser Ala Pro Val Glu Asp Asp Ser Asp Ala
                85                  90                  95
Glu Thr Tyr Gly Glu Glu Asn Asp Glu Gln Gly Asn Tyr Ser Lys Arg
            100                 105                 110

Lys Ile Val Ser Asn Trp Asp Arg Tyr Gln Asp Ile Glu Lys Glu Val
        115                 120                 125
Asn Asn Glu Ser Gly Glu Ser Gln Arg Gly Thr Asp Phe Ser Val Leu
    130                 135                 140
Leu Ser Ser Ala Gly Asp Ser Phe Ser Gln Phe Arg Phe Ala Glu Glu
145                 150                 155                 160
Lys Glu Trp Asp Ser Glu Ala Ser Cys Pro Lys Gln Asn Ser Ala Phe
                165                 170                 175
Tyr Val Asp Ser Glu Leu Leu Val Arg Ala Leu Gln Glu Leu Pro Leu
            180                 185                 190
Cys Leu Arg Leu Asn Val Ala Ala Glu Leu Val Gln Gly Thr Val Pro
        195                 200                 205
Leu Glu Val Pro Gln Val Lys Pro Lys Arg Thr Asp Asp Gly Lys Gly
    210                 215                 220
Leu Gly Met Gln Leu Lys Gly Pro Leu Gly Pro Gly Gly Arg Gly Pro
225                 230                 235                 240
Ile Phe Glu Leu Lys Ser Val Ala Ala Gly Cys Pro Val Leu Leu Gly
                245                 250                 255
Lys Asp Asn Pro Ser Pro Gly Pro Ser Arg Asp Ser Gln Lys Pro Thr
            260                 265                 270
Ser Pro Leu Gln Ser Ala Gly Asp His Leu Glu Glu Glu Leu Asp Leu
        275                 280                 285
Leu Leu Asn Leu Asp Ala Pro Ile Lys Glu Gly Asp Asn Ile Leu Pro
    290                 295                 300
Asp Gln Thr Ser Gln Asp Leu Lys Ser Lys Glu Asp Gly Glu Val Val
305                 310                 315                 320
Gln Glu Glu Glu Val Cys Ala Lys Pro Ser Val Thr Glu Glu Lys Asn
                325                 330                 335
Met Glu Pro Glu Gln Pro Ser Thr Ser Lys Asn Val Thr Glu Glu Glu
            340                 345                 350
Leu Glu Asp Trp Leu Asp Ser Met Ile Ser
        355                 360
```

<210> 5
<211> 1356
<212> DNA
<213> Homo sapiens

<400> 5

```
atgactttta acagttttga aggatctaaa acttgtgtac ctgcagacat caataaggaa 60
gaagaatttg tagaagagtt taatagatta aaaacttttg ctaattttcc aagtggtagt 120
cctgtttcag catcaacact ggcacgagca gggtttcttt atactggtga aggagatacc 180
gtgcggtgct ttagttgtca tgcagctgta gatagatggc aatatggaga ctcagcagtt 240
ggaagacaca ggaaagtatc cccaaattgc agatttatca acggctttta tcttgaaaat 300
agtgccacgc agtctacaaa ttctggtatc cagaatggtc agtacaaagt tgaaaactat 360
ctgggaagca gagatcattt tgccttagac aggccatctg agacacatgc agactatctt 420
ttgagaactg ggcaggttgt agatatatca gacaccatat acccgaggaa ccctgccatg 480
tatagtgaag aagctagatt aaagtccttt cagaactggc cagactatgc tcacctaacc 540
ccaagagagt tagcaagtgc tggactctac tacacaggta ttggtgacca agtgcagtgc 600
ttttgttgtg gtggaaaact gaaaaattgg gaaccttgtg atcgtgcctg gtcagaacac 660
aggcgacact ttcctaattg cttctttgtt ttgggccgga atcttaatat tcgaagtgaa 720
tctgatgctg tgagttctga taggaatttc ccaaattcaa caaatcttcc aagaaatcca 780
tccatggcag attatgaagc acggatcttt acttttggga catggatata ctcagttaac 840
aaggagcagc ttgcaagagc tggattttat gctttaggtg aaggtgataa agtaaagtgc 900
tttcactgtg gaggagggct aactgattgg aagcccagtg aagacccttg ggaacaacat 960
gctaaatggt atccagggtg caaatatctg ttagaacaga agggacaaga atatataaac 1020
aatattcatt taactcattc acttgaggag tgtctggtaa gaactactga gaaaacacca 1080
tcactaacta gaagaattga tgataccatc ttccaaaatc ctatggtaca agaagctata 1140
cgaatggggt tcagtttcaa ggacattaag aaaataatgg aggaaaaaat tcagatatct 1200
gggagcaact ataaatcact tgaggttctg gttgcagatc tagtgaatgc tcagaaagac 1260
agtatgccag atgagtcaag tcagacttca ttacagaaag agattagtac tgaagagcag 1320

ctaaggcgcc tgcaagagga gaagcttatc gattga                           1356
```

<210> 6
<211> 451
<212> PRT
<213> Homo sapiens

<400> 6

```
Met Thr Phe Asn Ser Phe Glu Gly Ser Lys Thr Cys Val Pro Ala Asp
 1               5                  10                  15
Ile Asn Lys Glu Glu Glu Phe Val Glu Glu Phe Asn Arg Leu Lys Thr
            20                  25                  30
Phe Ala Asn Phe Pro Ser Gly Ser Pro Val Ser Ala Ser Thr Leu Ala
        35                  40                  45
Arg Ala Gly Phe Leu Tyr Thr Gly Glu Gly Asp Thr Val Arg Cys Phe
    50                  55                  60
Ser Cys His Ala Ala Val Asp Arg Trp Gln Tyr Gly Asp Ser Ala Val
65                  70                  75                  80
Gly Arg His Arg Lys Val Ser Pro Asn Cys Arg Phe Ile Asn Gly Phe
                85                  90                  95
Tyr Leu Glu Asn Ser Ala Thr Gln Ser Thr Asn Ser Gly Ile Gln Asn
            100                 105                 110
Gly Gln Tyr Lys Val Glu Asn Tyr Leu Gly Ser Arg Asp His Phe Ala
        115                 120                 125
Leu Asp Arg Pro Ser Glu Thr His Ala Asp Tyr Leu Leu Arg Thr Gly
    130                 135                 140
Gln Val Val Asp Ile Ser Asp Thr Ile Tyr Pro Arg Asn Pro Ala Met
145                 150                 155                 160
Tyr Ser Glu Glu Ala Arg Leu Lys Ser Phe Gln Asn Trp Pro Asp Tyr
                165                 170                 175
Ala His Leu Thr Pro Arg Glu Leu Ala Ser Ala Gly Leu Tyr Tyr Thr
            180                 185                 190
Gly Ile Gly Asp Gln Val Gln Cys Phe Cys Cys Gly Gly Lys Leu Lys
        195                 200                 205
Asn Trp Glu Pro Cys Asp Arg Ala Trp Ser Glu His Arg Arg His Phe
    210                 215                 220
Pro Asn Cys Phe Phe Val Leu Gly Arg Asn Leu Asn Ile Arg Ser Glu
225                 230                 235                 240
Ser Asp Ala Val Ser Ser Asp Arg Asn Phe Pro Asn Ser Thr Asn Leu
                245                 250                 255
Pro Arg Asn Pro Ser Met Ala Asp Tyr Glu Ala Arg Ile Phe Thr Phe
            260                 265                 270
Gly Thr Trp Ile Tyr Ser Val Asn Lys Glu Gln Leu Ala Arg Ala Gly
        275                 280                 285
Phe Tyr Ala Leu Gly Glu Gly Asp Lys Val Lys Cys Phe His Cys Gly
    290                 295                 300
Gly Gly Leu Thr Asp Trp Lys Pro Ser Glu Asp Pro Trp Glu Gln His
305                 310                 315                 320
Ala Lys Trp Tyr Pro Gly Cys Lys Tyr Leu Leu Glu Gln Lys Gly Gln
                325                 330                 335
Glu Tyr Ile Asn Asn Ile His Leu Thr His Ser Leu Glu Glu Cys Leu
            340                 345                 350
Val Arg Thr Thr Glu Lys Thr Pro Ser Leu Thr Arg Arg Ile Asp Asp
        355                 360                 365
Thr Ile Phe Gln Asn Pro Met Val Gln Glu Ala Ile Arg Met Gly Phe
    370                 375                 380
Ser Phe Lys Asp Ile Lys Lys Ile Met Glu Glu Lys Ile Gln Ile Ser
385                 390                 395                 400
Gly Ser Asn Tyr Lys Ser Leu Glu Val Leu Val Ala Asp Leu Val Asn
                405                 410                 415
Ala Gln Lys Asp Ser Met Pro Asp Glu Ser Ser Gln Thr Ser Leu Gln


            420                 425                 430
Lys Glu Ile Ser Thr Glu Glu Gln Leu Arg Arg Leu Gln Glu Glu Lys
        435                 440                 445
Leu Ile Asp
    450
```

<210> 7
<211> 573
<212> DNA
<213> Homo sapiens

<400> 7

```
atggcgcacg ctgggagaac agggtacgat aaccgggaga tagtgatgaa gtacatccat 60
tataagctgt cgcagagggg ctacgagtgg gatgccgcgg ggcctgcgct cagcccggtg 120
ccacctgtgg tccacctgac cctccgccag gccggcgacg acttctcccg ccgctaccgc 180
cgcgacttcg ccgagatgtc cagccagctg cacctgacgc ccttcaccgc gcggggacgc 240
tttgccacgg tggtggagga gctcttcagg gacggggtga actgggggag gattgtggcc 300
ttctttgagt tcggtggggt catgtgtgtg gagagcgtca accgggagat gtcgcccctg 360
gtggacaaca tcgccctgtg gatgactgag tacctgaacc ggcacctgca cacctggatc 420
caggataacg gaggctggga tgcctttgtg gaactgtacg gccccagcat gcggcctctg 480
tttgatttct cctggctgtc tctgaagact ctgctcagtt tggccctggt gggagcttgc 540
atcaccctgg gtgcctatct gagccacaag tga                              573
```

<210> 8
<211> 190
<212> PRT
<213> Homo sapiens

<400> 8

```
Met Ala His Ala Gly Arg Thr Gly Tyr Asp Asn Arg Glu Ile Val Met
 1               5                  10                  15
Lys Tyr Ile His Tyr Lys Leu Ser Gln Arg Gly Tyr Glu Trp Asp Ala
            20                  25                  30
Ala Gly Pro Ala Leu Ser Pro Val Pro Pro Val Val His Leu Thr Leu
        35                  40                  45
Arg Gln Ala Gly Asp Asp Phe Ser Arg Arg Tyr Arg Arg Asp Phe Ala
    50                  55                  60
Glu Met Ser Ser Gln Leu His Leu Thr Pro Phe Thr Ala Arg Gly Arg
65                  70                  75                  80
Phe Ala Thr Val Val Glu Glu Leu Phe Arg Asp Gly Val Asn Trp Gly
                85                  90                  95
Arg Ile Val Ala Phe Phe Glu Phe Gly Gly Val Met Cys Val Glu Ser
            100                 105                 110
Val Asn Arg Glu Met Ser Pro Leu Val Asp Asn Ile Ala Leu Trp Met
        115                 120                 125
Thr Glu Tyr Leu Asn Arg His Leu His Thr Trp Ile Gln Asp Asn Gly
    130                 135                 140
Gly Trp Asp Ala Phe Val Glu Leu Tyr Gly Pro Ser Met Arg Pro Leu
145                 150                 155                 160
Phe Asp Phe Ser Trp Leu Ser Leu Lys Thr Leu Leu Ser Leu Ala Leu
                165                 170                 175
Val Gly Ala Cys Ile Thr Leu Gly Ala Tyr Leu Ser His Lys
            180                 185                 190
```

<210> 9
<211> 1476
<212> DNA
<213> Homo sapiens

<400> 9

```
atgtgcaata ccaacatgtc tgtacctact gatggtgctg taaccacctc acagattcca 60

gcttcggaac aagagaccct ggttagacca aagccattgc ttttgaagtt attaaagtct 120
gttggtgcac aaaaagacac ttatactatg aaagaggttc ttttttatct tggccagtat 180
attatgacta aacgattata tgatgagaag caacaacata ttgtatattg ttcaaatgat 240
cttctaggag atttgtttgg cgtgccaagc ttctctgtga aagagcacag gaaaatatat 300
accatgatct acaggaactt ggtagtagtc aatcagcagg aatcatcgga ctcaggtaca 360
tctgtgagtg agaacaggtg tcaccttgaa ggtgggagtg atcaaaagga ccttgtacaa 420
gagcttcagg aagagaaacc ttcatcttca catttggttt ctagaccatc tacctcatct 480
agaaggagag caattagtga gacagaagaa aattcagatg aattatctgg tgaacgacaa 540
agaaaacgcc acaaatctga tagtatttcc ctttcctttg atgaaagcct ggctctgtgt 600
gtaataaggg agatatgttg tgaaagaagc agtagcagtg aatctacagg gacgccatcg 660
aatccggatc ttgatgctgg tgtaagtgaa cattcaggtg attggttgga tcaggattca 720
gtttcagatc agtttagtgt agaatttgaa gttgaatctc tcgactcaga agattatagc 780
cttagtgaag aaggacaaga actctcagat gaagatgatg aggtatatca agttactgtg 840
tatcaggcag gggagagtga tacagattca tttgaagaag atcctgaaat ttccttagct 900
gactattgga aatgcacttc atgcaatgaa atgaatcccc cccttccatc acattgcaac 960
agatgttggg cccttcgtga gaattggctt cctgaagata aagggaaaga taaaggggaa 1020
atctctgaga aagccaaact ggaaaactca acacaagctg aagagggctt tgatgttcct 1080
gattgtaaaa aaactatagt gaatgattcc agagagtcat gtgttgagga aaatgatgat 1140
aaaattacac aagcttcaca atcacaagaa agtgaagact attctcagcc atcaacttct 1200
agtagcatta tttatagcag ccaagaagat gtgaaagagt ttgaaaggga agaaacccaa 1260
gacaaagaag agagtgtgga atctagtttg ccccttaatg ccattgaacc ttgtgtgatt 1320
tgtcaaggtc gacctaaaaa tggttgcatt gtccatggca aaacaggaca tcttatggcc 1380
tgctttacat gtgcaaagaa gctaaagaaa aggaataagc cctgcccagt atgtagacaa 1440
ccaattcaaa tgattgtgct aacttatttc ccctag                            1476
```

<210> 10
<211> 491
<212> PRT
<213> Mus musculus

<400> 2

```
Met Cys Asn Thr Asn Met Ser Val Pro Thr Asp Gly Ala Val Thr Thr
 1               5                  10                  15
Ser Gln Ile Pro Ala Ser Glu Gln Glu Thr Leu Val Arg Pro Lys Pro
            20                  25                  30
Leu Leu Leu Lys Leu Leu Lys Ser Val Gly Ala Gln Lys Asp Thr Tyr
        35                  40                  45
Thr Met Lys Glu Val Leu Phe Tyr Leu Gly Gln Tyr Ile Met Thr Lys
    50                  55                  60
Arg Leu Tyr Asp Glu Lys Gln Gln His Ile Val Tyr Cys Ser Asn Asp
65                  70                  75                  80
Leu Leu Gly Asp Leu Phe Gly Val Pro Ser Phe Ser Val Lys Glu His
                85                  90                  95
Arg Lys Ile Tyr Thr Met Ile Tyr Arg Asn Leu Val Val Val Asn Gln
            100                 105                 110
Gln Glu Ser Ser Asp Ser Gly Thr Ser Val Ser Glu Asn Arg Cys His
        115                 120                 125
Leu Glu Gly Gly Ser Asp Gln Lys Asp Leu Val Gln Glu Leu Gln Glu
    130                 135                 140
Glu Lys Pro Ser Ser Ser His Leu Val Ser Arg Pro Ser Thr Ser Ser
145                 150                 155                 160
Arg Arg Arg Ala Ile Ser Glu Thr Glu Glu Asn Ser Asp Glu Leu Ser
                165                 170                 175
Gly Glu Arg Gln Arg Lys Arg His Lys Ser Asp Ser Ile Ser Leu Ser
            180                 185                 190
Phe Asp Glu Ser Leu Ala Leu Cys Val Ile Arg Glu Ile Cys Cys Glu
        195                 200                 205
Arg Ser Ser Ser Ser Glu Ser Thr Gly Thr Pro Ser Asn Pro Asp Leu
    210                 215                 220
Asp Ala Gly Val Ser Glu His Ser Gly Asp Trp Leu Asp Gln Asp Ser
225                 230                 235                 240
```

```
Val Ser Asp Gln Phe Ser Val Glu Phe Glu Val Glu Ser Leu Asp Ser
                245                 250                 255
Glu Asp Tyr Ser Leu Ser Glu Glu Gly Gln Glu Leu Ser Asp Glu Asp
            260                 265                 270
Asp Glu Val Tyr Gln Val Thr Val Tyr Gln Ala Gly Glu Ser Asp Thr
        275                 280                 285
Asp Ser Phe Glu Glu Asp Pro Glu Ile Ser Leu Ala Asp Tyr Trp Lys
    290                 295                 300
Cys Thr Ser Cys Asn Glu Met Asn Pro Pro Leu Pro Ser His Cys Asn
305                 310                 315                 320
Arg Cys Trp Ala Leu Arg Glu Asn Trp Leu Pro Glu Asp Lys Gly Lys
                325                 330                 335
Asp Lys Gly Glu Ile Ser Glu Lys Ala Lys Leu Glu Asn Ser Thr Gln
            340                 345                 350
Ala Glu Glu Gly Phe Asp Val Pro Asp Cys Lys Lys Thr Ile Val Asn
        355                 360                 365
Asp Ser Arg Glu Ser Cys Val Glu Glu Asn Asp Asp Lys Ile Thr Gln
    370                 375                 380
Ala Ser Gln Ser Gln Glu Ser Glu Asp Tyr Ser Gln Pro Ser Thr Ser
385                 390                 395                 400
Ser Ser Ile Ile Tyr Ser Ser Gln Glu Asp Val Lys Glu Phe Glu Arg
                405                 410                 415
Glu Glu Thr Gln Asp Lys Glu Glu Ser Val Glu Ser Ser Leu Pro Leu
            420                 425                 430
Asn Ala Ile Glu Pro Cys Val Ile Cys Gln Gly Arg Pro Lys Asn Gly
        435                 440                 445
Cys Ile Val His Gly Lys Thr Gly His Leu Met Ala Cys Phe Thr Cys
    450                 455                 460
Ala Lys Lys Leu Lys Lys Arg Asn Lys Pro Cys Pro Val Cys Arg Gln
465                 470                 475                 480
Pro Ile Gln Met Ile Val Leu Thr Tyr Phe Pro
                485                 490
```

<210> 11
<211> 513
<212> DNA
<213> Homo sapiens

<400> 11

```
atgtctcaga gcaaccggga gctggtggtt gactttctct cctacaagct ttcccagaaa 60
ggatacagct ggagtcagtt tagtgatgtg gaagagaaca ggactgaggc cccagaaggg 120
actgaatcgg agatggagac ccccagtgcc atcaatggca acccatcctg gcacctggca 180
gacagccccg cggtgaatgg agccactggc cacagcagca gtttggatgc ccgggaggtg 240
atccccatgg cagcagtaaa gcaagcgctg agggaggcag gcgacgagtt tgaactgcgg 300
taccggcggg cattcagtga cctgacatcc cagctccaca tcaccccagg gacagcatat 360
cagagctttg aacaggatac ttttgtggaa ctctatggga acaatgcagc agccgagagc 420
cgaaagggcc aggaacgctt caaccgctgg ttcctgacgg gcatgactgt ggccggcgtg 480
gttctgctgg gctcactctt cagtcggaaa tga                              513
```

<210> 12
<211> 233
<212> PRT
<213> Homo sapiens

<400> 12

```
Met Ser Gln Ser Asn Arg Glu Leu Val Val Asp Phe Leu Ser Tyr Lys
 1               5                  10                  15
Leu Ser Gln Lys Gly Tyr Ser Trp Ser Gln Phe Ser Asp Val Glu Glu
            20                  25                  30
Asn Arg Thr Glu Ala Pro Glu Gly Thr Glu Ser Glu Met Glu Thr Pro
        35                  40                  45

Ser Ala Ile Asn Gly Asn Pro Ser Trp His Leu Ala Asp Ser Pro Ala
    50                  55                  60
Val Asn Gly Ala Thr Gly His Ser Ser Ser Leu Asp Ala Arg Glu Val
65                  70                  75                  80
Ile Pro Met Ala Ala Val Lys Gln Ala Leu Arg Glu Ala Gly Asp Glu
                85                  90                  95
Phe Glu Leu Arg Tyr Arg Arg Ala Phe Ser Asp Leu Thr Ser Gln Leu
            100                 105                 110
His Ile Thr Pro Gly Thr Ala Tyr Gln Ser Phe Glu Gln Val Val Asn
        115                 120                 125
Glu Leu Phe Arg Asp Gly Val Asn Trp Gly Arg Ile Val Ala Phe Phe
    130                 135                 140
Ser Phe Gly Gly Ala Leu Cys Val Glu Ser Val Asp Lys Glu Met Gln
145                 150                 155                 160
Val Leu Val Ser Arg Ile Ala Ala Trp Met Ala Thr Tyr Leu Asn Asp
                165                 170                 175
His Leu Glu Pro Trp Ile Gln Glu Asn Gly Gly Trp Asp Thr Phe Val
            180                 185                 190
Glu Leu Tyr Gly Asn Asn Ala Ala Ala Glu Ser Arg Lys Gly Gln Glu
        195                 200                 205
Arg Phe Asn Arg Trp Phe Leu Thr Gly Met Thr Val Ala Gly Val Val
    210                 215                 220
Leu Leu Gly Ser Leu Phe Ser Arg Lys
225                 230
```

## Description

### Field of the Invention

The present invention relates to methods for achieving high viable cell density and extended culture longevity in fed batch cell culture by using a high glucose feed. The methods are useful for increasing production of secreted proteins of interest.

### Background of the Invention

Mammalian cell culture is the system of choice for many recombinant protein production processes due to its ability to produce proteins with proper post-translational modifications. With increasing manufacturing demand, there is a strong motivation to improve process efficiency by increasing product yield. Attaining grams per liter production levels of biotherapeutics or other proteins in commercial production processes relies upon the optimization of both mammalian cell culture and engineering methods. Inherent in current high density, protein-free mammalian cell cultures is the problem of cell death of which apoptosis can account for up to 80% in a typical fed-batch bioreactor, induced in response to conditions such as nutrient and growth factor deprivation, oxygen depletion, toxin accumulation, and shear stress (Goswami et al., Biotechnol Bioeng 62:632-640 (1999)). Apoptosis limits the maximum viable cell density, accelerates the onset of the death phase and potentially decreases heterologous protein yield (Chiang and Sisk, Biotechnol Bioeng 91:779-792 (2005); Figueroa et al., Biotechnol Bioeng. 73:211-222 (2001), Metab Eng 5:230-245 (2003), Biotechnol Bioeng 85:589-600 (2004); Mercille and Massie, Biotechnol Bioeng 44:1140-1154 (1994)).

Apoptosis is a result of a complex network of signaling pathways initiating from both inside and outside the cell, culminating in the activation of cysteine aspartate proteases (caspases) that mediate the final stages of cell death. See Fig. 1. Various methods of apoptosis prevention have been used to maintain cell viability during extended production runs in mammalian cell culture (Arden and Betenbaugh, Trends Biotechnol 22:174-180 (2004); Vives et al., Metab Eng 5:124-132 (2003)). Altering the extracellular environment through media supplementation of growth factors, hydrolysates, and limiting nutrients has led to increased protein production and decreased apoptosis (Burteau et al., In Vitro Cell Dev Biol Anim. 39:291-296 (2003); Zhang and Robinson, Cytotechnology 48: 59-74 (2005)). Other researchers have turned to chemical and genetic strategies to inhibit the apoptotic signaling cascade from within the cell (Sauerwald et al., Biotechnol Bioeng 77:704-716 (2002), Biotechnol Bioeng 81:329-340 (2003]).

Researchers have found that over-expression of genes found upregulated in cancer cells can prolong viability in cells grown in bioreactors by preventing apoptosis upstream of caspase activation (Goswami et al., supra; Mastrangelo et al., Trends Biotechnol 16:88-95 (1998); Meents et al., Biotechnol Bioeng 80:706-716 (2002); Tey et al., J Biotechnol 79:147-159 (2000) and Biotechnol Bioeng 68:31-43 (2000). Upregulation of these proteins in production cell lines effectively suppressed apoptotic signaling within the cell, thereby limiting cell death in order to maintain viability and increase biotherapeutic production in some cases.

Also inherent in high density, protein-free mammalian cell cultures is the problem of waste accumulation and its detrimental effect on cell growth. The two most common cell culture waste products are lactate and ammonia. Numerous strategies have been devised to address the accumulation of excessive lactate build-up including 1) maintaining low medium glucose concentrations (Kurokawa et al., BiotechnolBioeng 44:95-103 (1994); Xie and Wang, Biotechnol Bioeng 43:1175-1189 (1993); Zhang et al., J Chem Technol Biotechnol 79:171-181 (2004); Zhou et al., Biotechnol Bioeng 46:579-587 (1995)); 2) feeding alternative sugars, including fructose (Martinelle et al., Biotechnol Bioeng 60:508-517 (1998), Altamirano et al., J Biotechnol 110:171-179 (2004) and J Biotechnol 125:547-556 (2006), Walschin and Wu, J Biotechnol 131:168-176 (2007); 3) partially knocking out lactate dehydrogenase (LDH) expression by homologous recombination or siRNA technology; 4) over-expression of pyruvate carboxylase; 5) use of dichloracetate (DCA), a pyruvate dehydrogenase (PDH) activator (via PDH kinase inhibition); 6) oxamic acid, an LDH competitive inhibitor; and 7) removal through perfusion (US Pat. Appl. Publ. No. 2009/0042253 A1).

Originally, the exclusive function of many of the apoptotic pathway proteins was believed to be binding at the mitochondrial membrane and regulating apoptosis through modulation of mitochondria permeability. Recent findings have shown that key proteins involved in apoptotic signaling interact with and have effects on proteins that control metabolism and energy homeostasis in the cell. See Majors et al., Metab Eng 9:317-326 (2007) for a review; and White et al., Nat Cell Biol 7:1021-1028 (2005). In a recent study, microarray analysis of CHO cells over-expressing Bcl-XL show that lactate dehydrogenase, a key enzyme in gluconeogenesis, is up-regulated.

Certain cells and viruses produce anti-apoptotic genes that function in the mitochondrial apoptotic pathway. These can be divided into three groups, namely 1) those that act early in the pathway, e.g., members of the Bcl-2 family of proteins; 2) those that act mid-pathway to disrupt or inhibit the apoptosome complex, e.g., Aven and 3) those that act late in the pathway, e.g., caspase inhibitors such as XIAP. The functionality of the majority of these genes have been studied by over-expressing them in mammalian expression systems, and in some cases the effect of combined over-expression of two or more genes, each derived from a different part of the pathway has been determined. Examples include 1) the additive effect of Bcl-XL and a deletion mutant of XIAP (XIAPΔ) in CHO cells (Figueroa et al., Metab. Eng. 5:230-245 (2003)); 2) E1B-19K and Aven in BHK cells (Nivitchanyong et al., Biotechnol Bioeng 98:825-841 (2007)) and 3) Bcl-XL, Aven and XIAPΔ (Sauerwald et al., supra, (2003); Sauerwald et al, Biotechnol Bioeng 94:362-369 (2006)). However, in these studies, the effect of the anti-apoptotic genes on the cellular metabolic state of the cell was not examined. Accordingly, a need exists in mammalian cell culture systems to optimize nutrient consumption and metabolite accumulation conditions to achieve increased viable cell density, longevity and productivity.

Figueroa et al., Biotechnol Bioeng 97(4):877-892 (2007) describe enhanced cell culture performance using inducible anti-apoptotic genes. US 2007/0092947 discloses compositions and methods for increasing the longevity of a cell culture. US 2008/0015164 discloses methods and compositions for inactivating dihydrofolate reductase genes, using fusion proteins comprising a zinc finger protein and a cleavage domain or cleavage half-domain. US 5,672,502 discloses fed-batch culture of animal cells comprising culturing the cells in nutrient medium characterized in that during the culture the medium is supplemented with a combined feed of one or more energy sources and one or more amino acids. Cory et al., Oncogene 22:8590-8607 (2003) summarises how proteins sense stress, interact with their relatives, perturb organelles such as the mitochondrion and endoplasmic reticulum and govern pathways to caspase activation. Yang et al., Science 275:1129-1132 (1997) describes one possible role of Bcl-2 in prevention of apoptosis, which is to block cytochrome c release from mitochondria. US 2005/0089993 discloses a variety of microscale bioreactors (microfermentors) and microscale bioreactor arrays for use in culturing cells.

## Brief Description of the Drawings

Fig. 1 shows mitochondrial apoptosis pathway components.

Figs. 2A-2C show the characterization of apoptotic$^R$ cell lines.

Fig. 3 shows confirmation of apoptotic$^R$ in EA167 and EAX197 by analysis by flow cytometry.

Figs. 4A-4C show growth profiles of apoptotic$^R$ cell lines generated from transfection of E1B-19K, E1B-19K+Aven and E1B-19K + Aven + XIAPΔ. Shake flask batch cultures of control (●), E64 (♦), EA167 (■) and EAX197 (▼) were monitored for viable cell density and viability.

Figs. 5A-5C show metabolite profiles of apoptotic$^R$ cell lines generated from transfection of E1B-19K + AVEN ± XIAPΔ. Shake flask batch cultures of control (●), EA167 (■) and EAX197 (▼) cell lines were monitored for various metabolites.

Figs. 6A and 6B show daily lactate replenishment of apoptotic cell lines. A) (●), control and EA167: □, un-fed and ■, lactate-fed) and B) EAX197 (Δ, un-fed and ▼, lactate-fed).

Figs. 7A-7G show growth and metabolic profiles of control and apoptotic$^R$ cell lines EA167 and EAX197 in the presence or absence of lactate feeding. Control (●), EA167 (□, un-fed and ■, lactate-fed), EAX197 (▲, un-fed and ▼, lactate-fed).

Figs. 8A-8G show profiles of amino acids depleted by apoptotic$^R$ cell lines upon lactate feeding. Control (●) EA167 (□, un-fed and ■, lactate-fed), EAX197 (▲, un-fed and ▼, lactate-fed).

Figs. 9A-9D show growth profiles and lactate accumulation profiles of control and apoptotic$^R$ cell lines EAX 197 cultured in high concentrations of glucose in a custom medium formulation. Apoptotic$^R$ cell line EAX197 (■) ; control cell line (♦).

Figs. 10A and 10B show antibody titers of cell lines generated from EAX197 and control host cell lines cultured in custom media formulation containing 30mM or 60mM glucose. Apoptotic$^R$ cell line EAX197 (■) ; control cell lines (□).

Figs. 11A and 11B show VCD and titer of a cell line containing the apoptotic$^R$ gene Bcl-2 with high and low cell seeding densities and media containing 30mM or 60mM glucose.

## Summary of the Invention

The invention provides a method for obtaining high viable cell density in a fed batch eukaryotic cell culture comprising the steps of: (a) culturing a eukaryotic cell line expressing one or more heterologous apoptotic-resistant (apoptotic$^R$) genes, wherein the apoptotic$^R$ genes are ElBl9K and AVEN, and one or more genes of interest; and (b) maintaining a high glucose media feed of about 60mM glucose during the exponential and stationary phases of the cell culture.

## Summary of the Disclosure

Disclosed herein is a method for obtaining high viable cell density in a fed batch eukaryotic cell culture comprising the steps of:

a) culturing a eukaryotic cell line expressing one or more heterologous apoptotic-resistant (apoptotic$^R$) genes and one or more genes of interest; and
b) maintaining a high glucose media feed during the exponential and stationary phases of the cell culture.

Also disclosed herein is a method of increasing production of secreted proteins in a fed batch eukaryotic cell culture comprising the steps of:

a) culturing a eukaryotic cell line expressing one or more heterologous apoptotic-resistant (apoptotic$^R$) genes and one or more genes of interest; and
b) maintaining a high glucose media feed during the exponential and stationary phases of the cell culture.

## Detailed Description

As used herein, the term "fed batch cell culture" means a cell culture process which is based on feeding of a growth limiting nutrient substrate to the culture. The fed-batch strategy is typically used in bio-industrial processes to reach a high cell density in a bioreactor. However, the addition of a nutrient such as glucose results in formation of metabolic waste products such as lactate and ammonia. Concentrations of 18mM lactate (Kurano et al., 1990) and 8mM ammonia (Hansen and Emborg, 1994) have been reported as inhibitory for eukaryotic cell growth.

Numerous strategies have been devised to address the accumulation of excessive lactate build-up in batch-fed cell culture and are discussed above. In the present invention, an alternative approach to reducing lactate concentration and increasing viable cell density and viability is presented. The method disclosed herein involves over-expressing one or more anti-apoptotic genes in a host cell line. The resultant apoptotic-resistant cell lines stimulate mitochondrial respiration and accumulate less lactate. Therefore, these cell lines can thrive when fed with high concentrations of glucose. Consequently, cultures of these apoptotic-resistant cell lines can reach high viable cell density. These cell lines also possess extended longevity due to their apoptotic-resistant nature.

The methods of the invention are useful for increasing viable cell density and viability in fed batch cell culture, such as Chinese Hamster Ovary (CHO), myeloma or hybridoma cell cultures. In particular, the methods of the invention are useful for increasing the integrated viable cell count (IVCC) of CHO cell cultures. The method disclosed herein comprises the steps of culturing a cell line expressing one or more heterologous apoptotic-resistant (apoptotic$^R$) genes and one or more genes of interest; and maintaining a high glucose media feed during the exponential and stationary phases of the cell culture. These lines are superior hosts for the development of production cell lines expressing proteins of interest such as peptides, peptide fusions, growth factors, hormones, antibodies, designed ankyrin repeat proteins (DARPins) and other polypeptides useful for therapeutic, diagnostic or research purposes. CHO cell lines useful in the method of the invention include CHO-K1 (Invitrogen, Carlsbad, CA) and CHOK1SV (Lonza Biologics, Slough, UK). Myeloma lines useful in the method of the invention include NS0 and Sp2/0.

The cell lines useful in the method of the invention express one or more heterologous anti-apoptosis genes. In particular, the genes encoding E1B19K (SEQ ID NOs: 1 and 2) and Aven (SEQ ID NOs: 3 and 4) are useful. The gene encoding XIAPΔ (SEQ ID NOs: 5 and 6) can also be used. These anti-apoptotic genes are representatives of the early, mid- and late stages of the apoptotic signaling pathway, respectively. Further, the gene encoding Bcl-2Δ can also be used (SEQ ID NOs: 7 and 8). Expression of the anti-apoptosis genes can be achieved by transfection techniques known to those skilled in the art. It is contemplated that other anti-apoptosis genes from these stages of the apoptotic signaling pathway, such as MDM2 (SEQ ID NOs: 9 and 10) and Bcl-XL (SEQ ID NOs: 11 and 12), would also be useful in the methods of the invention.

In the present invention, the use of cell lines expressing heterologous anti-apoptotic genes allows these cell lines to secrete less lactate or alternatively, to consume accumulated lactate, to reach integrated viable cell count (IVCC) values about two-fold higher than control cell lines while a high glucose feed is maintained in the culture. By increasing the viable cell densities of production cell lines in culture, the yields of products obtained from a bioreactor run are increased. Such enhanced productivities can result in lower production costs for complex biologics and at the same time, generate product of superior quality due to the absence of cell lysis of the non-viable cells, as lysed cells release proteases that degrade product. Accordingly, these lines are superior hosts for the development of production cell lines expressing a protein or proteins of interest.

In the method of the invention, the ability of the apoptotic$^R$ cell lines to consume or produce less lactate that would otherwise accumulate as waste provides for culture strategies to allow growth under conditions of high glucose as well as following glucose depletion. In different embodiments, the methods of the invention provide for increased peak viable cell density, increased longevity, , increased secreted protein titer, increased integrated viable cell count, reduced cellular calcium flux and increased mitochondrial membrane potential in batch fed cell culture. Furthermore, strategies of fed-batch cultivation that have been used to limit high levels of toxic lactate and ammonia are unlikely to be necessary for high productivity from these cell lines.

Following transfection, the site of chromosomal integration of an anti-apoptotic transgene can dictate the level of its expression. Moreover, when multiple anti-apoptotic genes are transfected, the level of expression of a particular anti-apoptotic transgene can impact the activity of other anti-apoptotic proteins the cell possesses since many of these proteins interact directly or indirectly to bring about physiological changes in the cell. Thus the quantitative contribution of each anti-apoptotic gene towards the overall apoptotic$^R$ characteristics of a cell line with multiple genes can be difficult to determine due to synergistic effects of the apoptotic$^R$ genes. Additionally, significant clone-to-clone variations exist with respect to apoptotic$^R$ characteristics even though all of them have been transfected with an identical set of transgenes. What is clear from the results presented in the Examples below is that each anti-apoptotic gene tested offered an incremental positive value towards improving the apoptosis$^R$ characteristics of the host cell line into which it was transfected. Within the above constraints, triple transfectants were generally superior to double transfectants, which in turn were superior to transfectants over-expressing only one anti-apoptotic transgene.

Consequently, following trsansfection with appropriate expression vectors, clones were obtained with variable levels of expression of E1B-19K, Aven, and XIAPΔ. The expression level of E1B-19K was relatively low and rare clones that had high expression level of E1B-19K were either unstable or had poor growth properties (data not shown). In the Examples below, only limited protection against cell death was observed in CHO cells expressing E1B-19K alone (Fig. 4).

Contrary to results obtained with cell lines expressing E1B-19K alone, data presented in the Examples below demonstrate that CHO K1 cell lines co-expressing Aven (EA167) or Aven and XIAPΔ (EAX197) exhibited improvements in viability, cell density and IVCC. The results presented in the Examples below further demonstrate that the expression of two anti-apoptosis genes in CHO K1 leads to a significant reduction in caspase activity and improved mitochondrial membrane potential in the presence of insults including staurosporine and extended batch cultures.

In the Examples below, the expression of a combination of E1B-19K, which is a functional homolog of Bcl-XL, plus Aven, and XIAPΔ was examined for effects on delaying cell death. The level of XIAPΔ expression in these transfected cells was not high relative to the endogenous wild type XIAP protein already present in the cell, perhaps due to the requirement for transfecting two plasmids in this system. Nonetheless, the addition of XIAPΔ in EAX197 (in addition to Aven and E1B-19K) provided a consistent enhancement in the maximum viable cell density in both unsupplemented (Figs. 5 and 7) and lactate supplemented cultures (Fig. 7). Indeed, the EAX197 cell line fed with lactate was able to sustain high viabilities at or above nearly 60% for 14 days, which was two days longer than the EA167 cells and four days longer than the control culture. The inclusion of XIAPΔ likely increases the cell's resistance to caspase activation and this is verified in Fig. 3 by the very low caspase activity of EAX197 even after treatment with staurosporine. Furthermore, the use of a mutant of XIAP (XIAPΔ) provides enhanced apoptosis resistance relative to the wild type XIAP protein. Thus, the protective capacity of the deletion mutant of XIAP (XIAPΔ) may be significant even if the expression of the protein is not high in these CHO lines.

In the Examples below, the role of one or more anti-apoptotic gene(s) in cell metabolism was investigated. Nutrient consumption and metabolite production were determined in the apoptotic$^R$ cell lines expressing E1B-19K in conjunction with Aven and/or XIAPΔ. In particular, the accumulation of the two most common cell culture waste products (i.e., lactate and ammonia) were compared in apoptotic$^R$ cell lines to that of the control cell line. In the data presented below, it was found that in shake-flask batch cultures in CD-CHO medium, on day 6-day 7 post-seeding, the control cell line accumulated 2.8g/L (31mM) lactate and 12mM ammonia (Fig. 5), which are well above the concentrations of 18mM of lactate (Kurano et al., supra) and 8mM of ammonia (Hansen and Emborg, supra) reported as inhibitory for cell growth. These levels may be part of the reason for loss in viability seen by day-8 post-inoculation in CHO control cell line (Fig. 4).

While the data presented below show that apoptotic$^R$ cell lines accumulate some lactate early, these cell lines began to consume lactate during the exponential phase and continued to increase in VCD well beyond that of the control cultures. Either the ability to consume lactate immediately following glucose exhaustion or an increase in apoptosis inhibition or both factors contributed to the continued cell growth. Furthermore, the apoptotic$^R$ cells only entered the stationary phase when the endogenous lactate was exhausted. In order to determine if the consumption of lactate was a general characteristic of these engineered cell lines, exogenous lactate was added to the apoptosis$^R$ cultures to replenish the depleted lactate (Fig. 6). The results presented in the Examples below show that the apoptotic$^R$ cultures consumed the added lactate and maintained high VCD and viabilities for four to seven additional days as compared to control cultures (for un-fed and lactate-fed cultures, respectively; Fig. 7A and B). An added process benefit is that lower levels of lactate in cultures of apoptotic$^R$ cell lines will result in lower osmolarity and higher pH, both of which are highly desirable for superior product quality.

Previous researchers have detected lactate consumption in hybridoma, NS0 myeloma and CHO cultures (deZengotita et al., 2000; Zhou et al., 1995 and 1997; Burky et al., 2007; Pascoe et al., 2007). However, these cells typically exhibit lactate production in the exponential phase and transition to consumption in the stationary phases to suggest that lactate can represent both an intermediate by-product and a carbohydrate fuel source (Burky et al., 2007; Brooks et al., 1985). In the results presented below, the control CHO cultures appeared to consume some lactate in the stationary phase, particularly in the 'high' glucose medium (Fig. 9), as seen in the previous studies. In contrast, the apoptotic$^R$ cells exhibited a dramatically different profile in which lactate was consumed during the exponential phase and the cells only entered the stationary phase when the exogenous lactate was depleted.

The conversion of pyruvate to lactate, catalyzed by lactate dehydrogenase (LDH), is reversible but strongly favors lactate formation with an equilibrium constant of $3.6x10^4$/M. However, when glycolysis is unable to keep up with tricarboxylic acid (TCA) demand, lactate can be converted back to pyruvate by one of the several isozymes of LDH favoring that reaction. The lactate consumed by apoptotic$^R$ cell lines likely feeds into the TCA cycle for cellular energetics and amino acid production. Interestingly, the ammonia profiles of the apoptotic$^R$ cultures differed from that of control cultures, with a repeated transitory drop in ammonia production for both the un-fed and lactate-fed cell lines as well as an overall decrease in ammonia production. The utilization of amino acids as an energy source in the TCA cycle is likely to lead to the accumulation of ammonia. Consequently, a reduction in ammonia production suggests that the apoptotic$^R$ cell lines may be obtaining a lower relative fraction of their TCA energy requirements from amino acids compared to the control cells, which is reasonable when considering that the apoptotic$^R$ cells are also consuming more lactate as a carbon source. It should be pointed out however that the drop in ammonia is transitory and the apoptotic$^R$ cells may still utilize amino acids as an energy source. Indeed, three amino acids, isoleucine, leucine and valine, all branched-chain amino acids were consumed more rapidly by apoptotic$^R$ cell lines and this consumption was exacerbated in lactate-fed cultures (Fig. 8A-C). Since the apoptotic$^R$ cell lines experienced continued growth relative to the control cell lines, these three amino acids may form building blocks for fatty acids and other proteins which are needed by the apoptotic$^R$ cells and also can be catabolized as a source of energy for the TCA cycle. A somewhat similar consumption profile is also observed for methionine (Fig. 8D).

In contrast to the amino acids which are observed to decline, a few amino acids were secreted including alanine, transiently and aspartate, transiently in the control cultures. A portion of the pyruvate can be converted to alanine by alanine amino-transferase. This pathway may be more active in apoptotic$^R$ cells leading to a larger rise in alanine concentration relative to the control cell lines (Fig. 8B). Aspartate, on the other hand, is the first member of the aspartate amino acid family and is formed along with alpha-ketoglurate by the conversion of TCA cycle intermediate oxaloacetate and glutamate using aspartate transminase. Its level increases only in the control cells for the first six days while being continuously depleted in the apoptotic$^R$ cell lines. Since this reaction is reversible, the depletion of aspartate in the apoptotic$^R$ cell lines early in the growth phase indicates a possible limitation in the reversible oxaloacetate product in the TCA cycle for apoptotic$^R$ cell lines. Given that oxaloacetate also reacts with Acetyl-CoA during the initial step of the TCA cycle, an increased flux of pyruvate into Acetyl-CoA from lactate in the apoptotic$^R$ cell lines may enhance the demand for oxaloacetate. In contrast, there may be less of a demand for Acetyl CoA in the control cell lines since they are not consuming lactate and thus they generate aspartate from oxaloacetate. Indeed, this lack of demand for Acetyl CoA could be driving the build-up of lactate as an alternative by-product to Acetyl CoA in the control cells over the first seven days of the cell culture. Alternatively, a lack of lactate consumption in the control cells could lead to a limitation in the supply of Acetyl-CoA and a concomitant increase in the production of aspartate.

These results are indicative of heightened TCA cycle energetics in cell lines expressing anti-apoptotic genes although an exact interpretation of the cellular reactions awaits more detailed metabolic flux analysis. Irrespective of the fate of the consumed lactate, in the present invention it has been demonstrated that apoptotic$^R$ cell lines consume accumulated lactate, which in turn, aids in their increased longevity and IVCC.

If apoptotic$^R$ cell lines are unique in that they can extend their longevity by consuming the accumulated lactate, then there is a possibility that these lines can be cultured in medium containing lactate or medium containing higher than standard concentrations of glucose. While growth and viability could not be supported in either type of cell line when lactate was provided as the sole carbon source, lactate consumption was observed in apoptotic$^R$ cell lines when it was provided as a component, in addition to glucose (data not shown) or when lactate accumulated as a waste product. This suggests that the apoptotic$^R$ cells cannot perform gluconeogenesis from pyruvate or obtain enough energy from lactate, but if glucose is provided in conjunction to lactate the cells can consume both the glucose as well as lactate accumulated or supplemented to the culture. Moreover, in medium containing high glucose (60mM), the IVCC (and VCD and viability, Figs. 9A-C) of apoptotic$^R$ cell lines was significantly higher than that of control cell lines. This is due to the fact that, in the high glucose medium, the control cell lines amassed more than 20mM lactate, which may have been toxic. The apoptotic$^R$ cell lines on the other hand, were consuming the lactate early in the growth phase and thus able to maintain higher numbers of viable cells that translated to an increase in IVCC of 170% over control (Fig. 9C).

Since the apoptotic$^R$ cell lines consumed lactate and achieved higher IVCC, production cell lines expressing proteins of interest such as therapeutic antibodies derived from apoptotic$^R$ host cell lines achieved significantly higher titers compared to production cell lines derived from control cell lines. Such higher titers illustrate the potential commercial benefits of using the anti-apoptosis genes in mammalian cell lines producing therapeutic or other proteins of interest.

The present invention will now be described with reference to the following specific, non-limiting examples.

## Examples

In the following Examples, CHO cell lines over-expressing anti-apoptosis genes were analyzed in shake flask cultures for peak viable cell density, longevity, caspase-3 activation and mitochondrial membrane potential (MMP). Additionally, nutrient consumption and metabolite production in these cell lines were compared to that of control cell lines to determine if the expression of anti-apoptosis genes had any effect on the nutrient consumption and metabolite accumulation in mammalian cell culture systems.

### Materials and Methods:

### Cell culture:

CHOK1SV cell line (Lonza Biologics, Slough, UK), designated as the Control cell line C1013A, was cultured in CD-CHO medium (Cat. No. 10743-011, Invitrogen, Carlsbad, CA), containing 30 mM Glucose and supplemented with 6mM L-Glutamine (Invitrogen Cat. No. 10313-021). In some instances, another animal protein-free medium containing various concentrations including 60mM glucose (defined as the high glucose medium) was used. Fetal Bovine serum was purchased from Hyclone Labs, Logan, UT (Cat. No. SH30071.03). Cell cultures were monitored by a Cedex automated cell counting instrument (Innovatis, Germany). Integrated viable cell count (IVCC, cell-day/ml) was calculated using the following formula:

$$IVCC\ (d1) = [VCD\ (d0) + VCD\ (d1)]/2 + VCD\ (d0)\ ,$$

where VCD = viable cell density

### Plasmid construction:

pBUDCE4.1 vector designed to constitutively express E1B-19K (EF-1a promoter), either alone or in conjunction with Aven (CMV promoter) and has been described (Nivitchanyong et. al., 2007). The vector expressing XIAPΔ (CMV promoter) was as described in Sauerwald et al., 2002. The blank vector refers to the original pBUDCE4.1 vector.

A model antibody (Ab #1) expression vector was constructed by cloning a Heavy and a Light chain cDNA into the Glutamine Synthase (GS) expression vector (obtained from Lonza Biologics, Slough, UK, under a research license).

### Generation of apoptotic$^R$ cell lines:

An exponential culture of CHOK1SV cell line was transfected with 1) pBUDCE4.1; 2) pBUDCE4.1-E1B-19K; 3) pBUDCE4.1-E1B-19K-Aven; and 4) pBUDCE4.1-E1B-19K-Aven and pCMV-XIAPΔ. Transfections were performed using Fugene (Roche, Cat. #1815075, Basel, Switzerland) according to the manufacturer's recommendation. Two days post-transfection, cells were plated in 96-well plates in growth medium containing 300μg/ml Zeocin (for transfections 1, 2 and 3 above); 300μg/ml Zeocin and 400μg/ml Hygromycin (for transfection #4), see Table 1. About 200 antibiotic resistant clones from each transfection were expanded and analyzed for caspase3/7 activity as described below. Promising clones underwent stability testing in absence of the respective antibiotics for ten passages.

**Table 1**

| Cell Line | Vector(s) transfected | Genes over-expressed | Clones selected by |
|---|---|---|---|
| Control | None | None | None |
| Blank | pBUDCE4.1 | None | Zeo |
| E64 | pBUDCE4.1-E1B-19K | E1B19K | Zeo |
| EA63 | pBUDCE4.1-E1B-19K-Aven | E1B-19K, Aven | Zeo |
| EA112 | | | |
| EA167 | | | |
| EA190 | | | |
| EAX64 | pBUDCE4.1-E1B-19K-Aven and pCMV-XIAPΔ | E1B-19K, Aven, XIAPΔ | Zeo, Hygro |
| EAX99 | | | |
| EAX148 | | | |
| EAX197 | | | |

Initially, a control cell line was developed by transfecting the blank vector into CHOK1SV cell line and selecting zeocin-resistant colonies. These cell lines had on average about 20% lower IVCC compared to the untransfected control CHOK1SV, hence CHOK1SV was subsequently used as the control cell line in all experiments. The most promising apoptotic$^R$ cell line, EAX197 as well as the control cell line were then used to develop production cell lines expressing a model antibody using the GS expression vector according to the manufacturer's recommendation. Antibody in cell culture was measured by Nephelometry (Beckman Array System).

**Shake-flask cultures of apoptotic$^R$ cell lines:**

Selected apoptotic$^R$ cell lines were cultured in un-fed batch mode in CD-CHO medium supplemented with 6mM Glutamine and the requisite antibiotic selection agent. In some experiments, the batch mode cultures of apoptotic$^R$ cell lines were fed with lactate to replenish the lactate they had depleted. Additionally, select Ab-expressing cell lines were cultured in a custom formulated animal protein-free medium supplemented with 6mM glutamine and 60mM glucose.

**Caspase 3/7 activity Assay:**

About $3x10^5$ cells of each clone were seeded in one ml of growth medium in a 24-well plate. On day 4 (d4) post seeding, about $1x10^5$ cells were transferred in triplicate to a 96well plate. Staurosprine (2$\mu$M fc) was added and the cells were incubated for 16h before assaying for caspase3/7 activity by APO-ONE kit (BD Labs). The procedure was repeated on d10, except that Staurosporine was omitted. The clones that had significantly lower caspase3/7 activity on both days were expanded into SF. The apoptotic$^R$ nature of the selected clones was confirmed by flow cytometry analysis (see below) and in some instances, by measuring the mitochondrial membrane potential.

**Analysis of apoptotic$^R$ clones by flow cytometry:**

About $1x10^6$ cells from exponential cultures were withdrawn from each shake flask into 24well plates, incubated with Staurosporine (2$\mu$M fc) for 16h, harvested and washed once in PBS. The cells were then incubated with CytoPerm (Cat. No. 2075KK, BD BioScience) to fix and permeablized them. Following a PBS wash, cells were incubated with FITC-labeled anti-caspase3 (Cat. No. 68654, BD BioScience) antibody before subjecting them to analysis by flow cytometry.

**Mitochondrial membrane potential (MMP) assay:**

Cells from shake flask cultures were withdrawn on d6 post-seeding and incubated with Staurosporine (5$\mu$M fc) for 2 hours. They were subsequently washed and processed with lipophilic cationic dye, JC-1 (Cayman Labs, Ann Arbor, MI) as per manufacturer's recommendation. Plates were read at FL535and FL595 and the ratio was calculated.

**Western blotting:**

About $1x10^7$ cells from indicated cell lines were harvested, washed in PBS and lysed in RIPA buffer containing 1% NP-40, 120mM Tris-Hcl, 150mM NaCl, 0.2mM PMSF and 1mM EDTA. Fifty micrograms of cytosolic protein from each sample was loaded on a 4-12% NuPAGE gradient gel and separated by SDS-PAGE in a Novex system (Invitrogen). Separated protein bands were transferred onto nitrocellulose and analyzed by using: 1) an anti-mouse antibody to human E1B-19K, 1:40 dilution, (Cat No. DP-17, CalBiochem, Gibbstown, MD); 2) an anti-Rabbit antibody to human Aven, 1:1,000 dilution, (Cat No. 612521, BD BioScience, San Jose, CA); and 3) an anti-mouse antibody to human XIAP (Cat No. 616713, BD BioScience). The above protocol, including the antibodies used, was optimized and standarized using known quantities (~10ng each) of purified E1B-19K, Aven and XIAP, purchased from commercial sources. The protein bands were visualized using ECL detection kit (GE Healthcare, Piscataway, NJ).

**Determination of metabolite concentrations in culture medium:**

Concentration of all key metabolites and waste products were determined using an YSI 2700 automated analyzer. Ammonium ion concentration was determined by flow injection analysis (Campmajo et al, 1994). Amino acids were measured by HPLC (Waters, Milford, MA), using a reversed-phase column (Waters).

**Example 1**

**Generation and characterization of apoptotic-resistant cell lines**

A list of cell lines used in this study, the expression plasmids that were transfected in each case to generate the cell lines and the selection agent(s) used for isolating the transfectomas is shown in Table 1 above. The cell lines listed are representative of multiple clones that were generated from each transfection. The name of each cell line was derived from the anti-apoptotic genes that were transfected into the host cell line. For example, EAX197 is a cell line that was transfected with E1B-19K (E), Aven (A) and XIAPΔ (X). The notation, apoptotic$^R$, will be used to refer to a cell line that has been transfected by one or more of these anti-apoptotic genes.

All apoptotic$^R$ cell lines used in this study were characterized by Western blotting, caspase 3/7 activity and mitochondrial membrane potential (data not shown). The single transfectant E64, or double transfectants EA63, EA112, EA167 and EA190, all expressed levels of E1B-91K that were above background levels observed in the control, untransfected cell line, C1013A. Of the four double transfectants, cell lines EA112 and EA167 expressed both E1B-19K (~19KDa) and Aven (~55 KDa). Cell lines EA63 and EA190 expressed significant levels of E1B-19K but only very low levels of Aven. Of the four cell lines that were generated by transfecting the dual vector pBUDCE4.1-E1B-19K-Aven as well as pCMV-XIAPΔ, namely, EAX64, EAX99, EAX148 and EAX197, only EAX197 expressed detectable levels of all three proteins.

An ~55kDa band was observed in all lanes including in the untransfected control lane, C1013A, above the recombinant Aven band was believed to be the endogenous Aven protein as it was visualized with post-immune but not with pre-immune sera (Sauerwald et al., 2002, Chau et al., 2000). The transfected Aven gene lacks the first six amino acids, which have been shown not to impair the biological activity of the protein. Consequently, transfected Aven is slightly smaller than its endogenous counterpart. A 40KDa band, which may represent a degradation product of Aven, was observed in all cell lines that over-expressed this protein in conjunction with E1B-19K. Degradative products of similar sizes were observed previously when Aven was over-expressed with Bcl-XL (Sauerwald et al., 2005) or Bcl-2 (Figueroa, unpublished observations). Additionally, the expression of Aven was significantly lower in triple transfectants, including EAX197 over-expressing E1B-19K, Aven and XIAPΔ.

Interestingly, the level of expression of transfected XIAPΔ was significantly lower compared to the endogenous levels of the intact XIAP protein. Note that the apparent difference in expression level between XIAP and XIAPΔ could be explained in part by the difference in antibody binding efficiency of these two forms of the anti-apoptotic protein. The apparent expression level difference between the endogenous XIAP and the transgenic XIAPΔ questions the contribution of this transfected protein in preventing apoptosis. Indeed this study suggests that there is not always a direct correlation between the expression level of a given anti-apoptotic gene and the level of apoptosis inhibition the protein can confer on a cell line.

Each apoptotic$^R$ cell line was examined for caspase 3/7 activity by APO-ONE as this activity provides an indication of the degree of resistance of a cell line to apoptosis. Briefly, in this assay, the profluorescent substrate Z-DEVD-R110 is cleaved by caspase 3/7 in a dose-dependent manner. Exponential cultures of EA167 and EAX197 as well as the control cell line were treated with staurosporine for 16 hours to induce apoptosis, following which caspase 3/7 was measured. The caspase 3/7 activity in staurosporine treated control cells was arbitrarily set at 100%. Under these conditions, the caspase activities of the EA167 and EAX197 cells were 30% and 35%, respectively, of the control, suggesting that these cell lines were at least partially resistant to apoptosis induction (Fig. 2A). As further evidence of the apoptotic$^R$ nature of EA167 and EAX197, staurosporine-treated cultures of these two cell lines and the control cell line, were labeled with FITC-conjugated anti-caspase 3 antibody followed by analysis by flow cytometry. As can be seen from Fig. 3, all three cell lines, whether untreated or vehicle (DMSO)-treated, had 1% or less caspase 3-positive population. However, 91% of the control cell population was positive for caspase 3 following staurosporine treatment. In contrast, only 9% of the population derived from cell line EAX197 and 30% of that derived from cell line EA167 were positive for caspase 3, confirming the apoptotic$^R$ phenotype of these two cell lines.

To examine if the resistance to apoptosis leads to increased longevity, multiple EA cell lines were grown in shake-flasks (batch mode) and both viable cell density (VCD) and integrated viable cell count (IVCC) were monitored. Increases in the integrated viable cell count improve the volumetric productivity of a culture, assuming that the specific productivity of the cell remains unaltered. Therefore IVCC can be used as a parameter for screening superior apoptotic$^R$ cell lines that can serve as hosts for the development of production cell lines. As shown in Fig. 2B, the cell lines with lower relative caspase 3/7 activity tended to generate cell lines with higher IVCC. Although each of the EA cell lines showed reduced caspase 3/7 activity relative to the control, there was no strict correlation between IVCC and the level of caspase 3/7 that each cell line possessed. Moreover, some apoptotic$^R$ cell lines were more stable than their sister clones with respect to the degree of anti-apoptotic phenotype. EA167 was chosen for further study because it possessed both potent anti-apoptotic activity and robust growth in shake flask cultures. Additionally, EA167 was the most stable cell line in this panel, expressing Aven at high levels as detected by Western blot.

Many of the signaling molecules in the apoptosis pathway interact with or reside within the mitochondria. There these proteins interact with the mitochondrial membrane and regulate apoptosis through modulation of the mitochondria permeability. Therefore, mitochondrial membrane potential (MMP) serves as another monitor of cell physiology in which to compare the characteristics of the apoptotic$^R$ nature of EA167 and EAX197 versus control. As seen in the assay results of Fig. 2C, both EA167 and EAX197 had higher MMP than the control cell line.

**Example 2**

**Effect of E1B-19K on viable cell density of CHOK1SV**

The growth profiles of double transfectants over-expressing E1B-19K in conjunction with Aven (EA167), triple transfectants over-expressing E1B-19K in conjunction with Aven and XIAPΔ (EAX197), as well as the transfectant over-expressing E1B-19K only (E64) are compared to the control (host) cell line in Fig. 4. While the peak VCD of the control cell line reached $7.7 \times 10^6$ cells/ml and the peak VCD of E64 (expressing E1B-19K only) was $8.9 \times 10^6$ cells/ml, that of EA167 was $1.4 \times 10^7$ cells/ml and that of EAX197 was more than $1.5 \times 10^7$ cells/ml, an increase of over 190% above the control cell line. The viability of EA167 and EAX197 showed a sharp decline by d8 or d9 post-seeding, presumably because of nutrient depletion, while the control and the E64 cell lines viability declined more slowly (Fig. 4B). The net effect on IVCC is shown in Fig. 4C. The IVCC of the control cell line was $4.9 \times 10^7$ cell-day/ml, followed by that of E64 ($5.2 \times 10^7$ cell-day/ml). Clearly, the IVCC of the two apoptotic$^R$ cell lines, EA167 ($6.3 \times 10^7$ cell-day/ml) and EAX197 ($6.6 \times 10^7$ cell-day/ml) were significantly higher (136% and 140% for EA167 and EAX197, respectively) than that of control cell lines.

In addition to EA167 and EAX197, several other apoptotic$^R$ lines exhibited similarly low lactate production phenotype (data not shown). Additionally, wild-type cell lines with low lactate production characteristics, though rare, have been reported (Pascoe et al, 2007).

**Example 3**

**Metabolic profile of apoptotic$^R$ cell lines**

In order to compare the physiology of the apoptotic$^R$ cell lines EA167 and EAX197 which could protect against apoptosis to the control cell line in batch cultures, the levels of key nutrients and amino acids that were provided at the beginning of the batch culture, namely, glucose, glutamate and glutamine, were monitored. Additionally, the accumulation of the waste products ammonia and lactate were also monitored. Shown in Fig. 5 are the levels of these metabolites when EAX197 and EA167 as well as control cell line were cultured in batch mode in shake-flasks in commercial CD-CHO medium. As shown in Fig. 5A, glucose is rapidly depleted from the medium of all three cell lines by day-6 or day-7 post-seeding. No significant differences in glutamine depletion were discernable between apoptotic$^R$ and control cell lines and glutamate levels did not vary greatly for the three cell lines across all days (data not shown). All three cell lines showed an accumulation of lactate during early cell growth phase up to day-4 (Fig. 5B). In the control cell line, lactate continued to increase up until day-6 (at which time it reached 2.8g/L) and then a slight decrease was observed with the lactate concentration eventually reaching 1.8g/L. This amount of lactate is expected to lower the pH of the culture and in bioreactors, where pH is controlled by the addition of bicarbonate, the osmolarity would be elevated. All these characteristics, in addition to other stress factors, could lead to a toxic environment for the animal cells. In contrast, the lactate levels in the apoptotic$^R$ cell lines increased for a shorter period and then dropped rapidly from day 5 onward with the lactate being completely eliminated by day 7 (EA167) or day 8 (EAX197). Interestingly, the time point at which the lactate dropped to zero immediately preceded the time at which the VCD dropped precipitously in Fig. 5A for these two cell lines, suggesting that the loss in cell viability was due to the absence of any available glucose or lactate in the medium.

Another difference observed between apoptotic$^R$ and control cell lines is the ammonia profile (Fig. 5C). The ammonia levels in EA167 and EAX197 declined slightly at day 6, at which time the decline in lactate had begun, and then ultimately increased again. However, the overall ammonia levels of the apoptotic$^R$ lines were lower than that of control lines, which was surprising since the apoptotic$^R$ lines reached higher IVCC and thus attained more overall cell growth compared to the control cell line (Fig. 4C).

From the above data, it is obvious that the characteristics of the apoptotic$^R$ cell lines are dramatically different from that of the control cell lines. However, when multiple clones are examined under somewhat different experimental conditions, a wide spectrum of phenotypes is observed among the apoptosis$^R$ transfectants. Since phenotypic variability exists in clonal isolates of the control cell line as well, it is possible to select rare apoptotic$^R$ clones from control cell line by utilizing a suitable screening regimen. In this respect, Mattanovich and Borth (2006) have reviewed the application of cell sorting to isolate rare variants with desirable properties from wild-type (control) cell line.

**Example 4**

**Effect of lactate feeding**

The amount of lactate present in a cell culture at any point is dependent on the amount of lactate secreted by that cell line less the amount consumed by it. In order to determine if the apoptotic$^R$ cell lines were actually consuming lactate or if the net lactate production was lower in these cell lines, cultures of all three cell lines were monitored daily and any major difference in lactate concentration between apoptotic$^R$ and control cell line was eliminated by the addition of exogenous lactate. As observed previously, the lactate level in the un-fed apoptotic$^R$ EA167 and EAX197 began to drop at day-5 (Figs. 6A and 6B) with exhaustion of lactate by day-7 (EA67, un-fed) or day-8 (EAX197, un-fed). The lactate profile of the control cell line continued to increase until d6 at which time the level declined slightly for the next three days but never dropped to zero. Upon daily monitoring of the apoptosis$^R$ culture, lactate was added to separate fed-batch cultures up to 2.5g/L.at day-7 (for EA167, Fig. 6A) and 2g/L at day-8 (for EAX197, Fig. 6B) in order for levels to be similar to that of the control cell line. Even with the addition of supplemental lactate, the lactate level dropped much more rapidly in the apoptotic$^R$ cultures compared than the controls from day-6 to day-12. Indeed, the lactate level dropped from 2.5 to 0.2g/L in the EA167 fed culture from day-6 to day-7 while the lactate decline was only from 2.7 to 2.4g/L in the control culture over the same time period. Thus, the lactate appears to be consumed in the apoptotic$^R$ cell lines at an accelerated rate compared to the control cell lines for at least 7 days following addition of supplemental lactate.

The effect of lactate supplementation on VCD and cell viability is shown in Figs. 7A and 7B, respectively. As previously observed (Fig. 5A), the VCD of the un-fed apoptotic$^R$ cell lines declined rapidly starting at day-8 (EA167) and day-9 (EAX197). This is presumably because one or more essential media components, including glucose and/or lactate, have been exhausted in these cultures. The control cell line exhibited a slow decline starting at day-7. However the lactate-supplemented cultures (EA167, lactate-fed and EAX197, lactate-fed) exhibited a much slower decline in VCD, and in one cell line (EAX197, lactate-fed), the VCD stabilized at between $8x10^6$ and $10x10^6$ cells/ml until d14 before this culture eventually succumbed. The cell viabilities shown in Fig. 7B exhibited a similar more gradual decline for the two apoptotic$^R$ cultures with supplemented lactate in the media. These results suggest that the apoptotic$^R$ cell lines are capable of utilizing both endogenous and supplemental lactate efficiently to retain their viable cell density and viability while the control cell line is unable to implement a similar efficient lactate consumption strategy. This difference is reflected in the IVCC of the lactate-fed apoptotic$^R$ cell lines, which were nearly 180-220% higher than the un-fed apoptotic$^R$ cell lines and about 235%-250% higher than the control cultures.

Most of the other nutrients and metabolites shown in Figs. 7D-F exhibited profiles similar to that observed previously in the un-fed cultures of Fig. 5. Glucose and glutamine were rapidly consumed at early time points in the culture. The ammonia profile of the apoptotic$^R$ cell lines once again exhibited a slight decline at the time at which lactate consumption began in both the unsupplemented and lactate-supplemented cultures. As in the previous experiments, the final ammonia level of the apoptotic$^R$ cell lines was below control levels even though the IVCC was considerably higher in all four apoptotic$^R$ cultures. The glutamate levels differed between lactate-fed and un-fed cultures with glutamate depletion being significantly increased in the fed culture as compared to the un-fed and control cell lines.

**Example 5**

**Amino acid profile of apoptotic$^R$ and control cell lines**

The concentrations of each of the twenty amino acids were monitored periodically in control cell lines as well as in un-fed and lactate-fed apoptotic$^R$ cell line cultures (Table II and Fig. 8). With respect to the consumption or production of amino acids, other than glutamine and glutamate, it should be first stated that none of the nine essential amino acids (phenylalanine, threonine, methionine, lysine, tryptophan, leucine, isoleucine, valine and histidine) were exhausted, as indicated in Table II. In apoptotic$^R$ (un-fed) and control cell line cultures, asparagine, serine, tryptophan and cysteine (highlighted in italics) were almost completely exhausted. Interestingly, all three branched-chain amino acids, namely, isoleucine, leucine and valine (shaded light grey in Table II) were more rapidly depleted from the culture medium by apoptotic$^R$ cell lines compared to the control, and this depletion is more pronounced in lactate-fed cultures (Figs. 8A-D). In addition to glutamate, the final levels of these three amino acids were much lower from the medium in lactate-fed cultures. Other amino acids with a high percentage consumed (final concentration below 50% of their initial concentration in the medium) included tyrosine, phenylalanine, methionine (Figure 8D) and aspartic acid (Fig. 8E).

A comparison of the amino acids that were excreted into the culture medium between the control and the apoptotic$^R$ cell lines is also of interest to consider. As shown in Fig. 8E, the control cell lines accumulated aspartic acid in the culture medium over the first six days while there was a decline in the aspartic acid levels over that same time period for all four apoptotic$^R$ cultures. Additionally, the apoptotic$^R$ cell lines secrete significantly more alanine (Fig. 8F) over the first 6 days as compared to the control cell line, while all the cell lines consume it at later times. Also, the apoptotic$^R$ cell lines produce more homocysteine over the entire cell culture in comparison to the control cell lines (Fig. 8G).

## Example 6

## Batch culture in the presence of high glucose

If the apoptotic$^R$ cell lines can utilize lactate, then there is a possibility that these lines may potentially thrive at glucose concentrations that could be inhibitory for control cells due to accumulation of toxic levels of lactate. Additionally, it is possible that growth of these lines could be supported by culture medium containing lactate as the sole carbon source. It was not possible to use the CD-CHO medium used in the previous experiments because such as high glucose would lead to unsustainable osmolarity and thus a new medium had to be utilized for these experiments. Therefore, we compared the growth kinetics of EAX197, which exhibited the highest sustained growth in previous experiments, to that of control cell lines in a custom-formulated animal protein-free medium containing 60mM glucose (high glucose) or 30mM lactate. Neither the apoptotic$^R$ nor the control cell lines exhibited sustained growth in the complete absence of glucose, i.e., in the presence of lactate only (data not shown). When both cell lines were cultured in the same custom-formulated medium containing 'high' (60mM) concentrations of glucose, EAX197 exhibited a higher VCD, viability and IVCC (Figs. 9A-C) than the control cell line especially at later times in the cell culture. EAX197 cell line exhibited a smaller overall increase in IVCC (130%) over control than in the previous CD-CHO medium but the difference was still significant due to increased viability at later times. The lactate profiles of these two cell lines cultivated in 'high' glucose are shown in Fig. 9D. As in the case with IVCC, the lactate profiles of both lines differed somewhat from that observed in CD-CHO medium (Fig. 5D). Consistent with previous results with the CD-CHO medium, the apoptotic$^R$ cell line in the custom formulation medium initiated net lactate consumption earlier (by day 2, as shown in Fig. 9D) than the control and the maximum lactate level was much lower (1.2g/L compared to 2.4g/L for the control cell line). The control cell exhibited a net production of lactate until day 5 at which point the control cells also began to consume the lactate in the medium. Since the CD-CHO medium is a commercial formulation with a propriety composition, it was not possible to compare exactly the medium component(s) in the two formulations in order to understand the cause of the different lactate consumption profiles in the different formulations.

## Example 7

## Productivity of an apoptotic$^R$ cell line in high glucose medium

The apoptotic$^R$ cell line, EAX197 along with the control cell line were subsequently used as hosts for the development of production cell lines expressing a model antibody. An equal number of clones were surveyed for each cell line and the best clone generated from each host was then compared in shake-flask-batch mode for Ab productivity. Custom formulated medium containing 60mM ('high') glucose as a carbon source was used in order to compare their performance in a high glucose environment. In the standard medium (Fig. 10A), the production cell line derived from EAX197 had titers of 489mg/L, which is 145% increase over the control cell line, which had a titer of 337mg/L. The same cell line in high glucose medium (Fig. 10B) had titers of 687mg/L, which is 178% increase over that of the control cell line (384mg/L). The enhanced production was due at least in part to a 188% increase in IVCC for EAX197 versus the control cell line (data not shown).

## Example 8

## Effect of over-expression of E1B19K+AVEN +/- XIAPΔ in blocking apoptosis

The major activators of the apoptosis cascade in mitochondria are shown in Fig. 1. In our attempt to develop an apoptotic-resistant CHO cell line, we selected three anti-apoptotic genes that act at early, mid- and late stage of apoptosis. These genes were E1B19K (E), AVEN (A) and XIAPΔ (X). CHOK1SV, the widely used transfection host cell line was transfected with E1B19K+AVEN with/without XIAPΔ. A large number of transfectomas were screened and a few clones from each transfection were selected for further study. Growth profile study of these cell lines show that they reach very high VCD and in general, their IVCC was about two-fold higher than that of control. The apoptotic$^R$ cell lines lost viability by day-8 post-seeding, but regained it day-10 onwards by consuming lactate. The culture fluid of apoptotic$^R$ lines expressing E1B19K+AVEN±XIAPΔ had reduced or undetectable levels of ammonia and lactate and depleted glutamate from the culture media.

**Example 9**

**Use of Bcl-2d to increase antibody titer**

The results above demonstrate that apoptotic$^R$ cell lines accumulate less lactate compared to control cells and viability of apoptotic$^R$ cultures can be maintained in high glucose medium. In the following shake-flask batch experiment, an apoptotic$^R$ cell line expressing Bcl-2d and antibody heavy and light chains (C2088B) was seeded at various seeding densities in standard (4.8g/L) and high concentration of glucose (9.5g/L). The results show that the apoptotic$^R$ cell line, when seeded at 1e6cells/ml in medium containing 9.5g/L glucose, reached peak VCD of 14e6cells/ml on day-7 post-seeding (Fig. 11A), and antibody titer of 2g/L. In comparison, C2088B cultured under the standard conditions (0.3e5cells/ml inoculation and 4.8g/L) had peak VCD of 7e6cells/ml and antibody titer of 1g/L (Fig. 11B). This translates to a 200% increase in antibody titers for high glucose condition as compared to standard conditions (i.e., seeding density of 0.3e6c/ml in medium containing 4.8g/L glucose).

## Claims

1. A method for obtaining high viable cell density in fed batch eukaryotic cell culture comprising the steps of:

   a) culturing a eukaryotic cell line expressing one or more heterologous apoptotic-resistant (apoptotic$^R$) genes, wherein the apoptotic$^R$ genes are E1B19K and AVEN, and one or more genes of interest; and
   b) maintaining a high glucose media feed of about 60mM glucose during the exponential and stationary phases of the cell culture.

2. The method of claim 1 wherein the eukaryotic cell line is:

   a) a Chinese Hamster Ovary (CHO) cell line, wherein the CHO cell line is optionally CHO-K1 or CHO-K1SV;
   b) a myeloma cell line, wherein the myeloma cell line is optionally NSO or Sp2/0; or
   c) a hybridoma.

3. The method of claim 1 wherein the apoptotic$^R$ genes further comprise XIAPΔ.

4. The method of claim 2a) wherein the CHO cell line consumes accumulated lactate during the cell culture exponential phase.

5. The method of claim 2a) wherein the CHO cell line secretes less lactate during the cell culture exponential phase than a CHO cell line not containing one or more apoptotic$^R$ genes.

6. The method of claim 1 wherein the peak viable cell density (VCD) is increased.

7. The method of claim 1 wherein the longevity of the cell culture is extended.

8. The method of claim 1 wherein the titer of the cell culture is increased.

9. The method of claim 1 wherein the integrated viable cell count (IVCC) of the cell culture is increased.

10. The method of claim 1 wherein the cellular calcium flux is reduced.

11. The method of claim 1 wherein the mitochondrial membrane potential is increased.

12. The method of claim 1 wherein the genes of interest encode an antibody heavy chain and an antibody light chain.

**Patentansprüche**

1. Verfahren zur Erzielung einer hohen Dichte lebensfähiger Zellen in einer Fed-Batch-Kultur eukaryontischer Zellen, umfassend die Schritte:

   a) Kultivieren einer ein oder mehrere heterologe Apoptoseresistenz(Apoptose$^R$) -Gene, wobei es sich bei den Apoptose$^R$-Genen um E1B19K und AVEN handelt, und ein oder mehrere interessierende Gene exprimierenden eukaryontischen Zelllinie; und
   b) kontinuierliches Zuführen eines Mediums mit einem hohem Glucoseanteil von etwa 60 mM Glucose während der exponentiellen und stationären Phasen der Zellkultur.

2. Verfahren nach Anspruch 1, wobei es sich bei der eukaryontischen Zelllinie um:

   a) eine CHO(Chinese Hamster Ovary)-Zelllinie, wobei es sich bei der CHO-Zelllinie wahlweise um CHO-K1 oder CHO-K1SV handelt,
   b) eine Myelomzelllinie, wobei es sich bei der Myelomzelllinie wahlweise um NS0 oder Sp2/0 handelt, oder
   c) ein Hybridom handelt.

3. Verfahren nach Anspruch 1, wobei die Apoptose$^R$-Gene ferner XIAPΔ umfassen.

4. Verfahren nach Anspruch 2a), wobei die CHO-Zelllinie angesammeltes Lactat während der exponentiellen Zellkulturphase verbraucht.

5. Verfahren nach Anspruch 2a), wobei die CHO-Zelllinie weniger Lactat während der exponentiellen Zellkulturphase sezerniert als eine nicht ein oder mehrere Apoptose$^R$-Gene enthaltende CHO-Zelllinie.

6. Verfahren nach Anspruch 1, wobei der VCD(Viable Cell Density)-Spitzenwert erhöht ist.

7. Verfahren nach Anspruch 1, wobei die Langlebigkeit der Zellkultur verlängert ist.

8. Verfahren nach Anspruch 1, wobei der Titer der Zellkultur erhöht ist.

9. Verfahren nach Anspruch 1, wobei der IVCC(Integrated Viable Cell Count)-Wert der Zellkultur erhöht ist.

10. Verfahren nach Anspruch 1, wobei der zelluläre Calciumfluss reduziert ist.

11. Verfahren nach Anspruch 1, wobei das mitochondriale Membranpotential erhöht ist.

12. Verfahren nach Anspruch 1, wobei die interessierenden Gene für eine schwere Antikörperkette und eine leichte Antikörperkette codieren.

**Revendications**

1. Procédé d’obtention d’une densité élevée de cellules viables dans une culture semi-discontinue de cellules eucaryotes comprenant les étapes de :

a) culture d'une lignée cellulaire eucaryote exprimant un ou plusieurs gènes de résistance à l'apoptose hétérologues (apoptotic$^R$), les gènes apoptotic$^R$ étant E1B19K et AVEN, et un ou plusieurs gènes d'intérêt ; et
b) maintien d'une alimentation de milieu riche en glucose à environ 60 mM de glucose pendant les phases exponentielle et stationnaire de la culture de cellules.

**2.** Procédé de la revendication 1 dans lequel la lignée cellulaire eucaryote est :

a) une lignée cellulaire d'ovaire de hamster chinois (CHO), la lignée cellulaire CHO étant facultativement CHO-KI ou CHO-KISV ;
b) une lignée cellulaire de myélome, la lignée cellulaire de myélome étant facultativement NSO ou Sp2/0 ; ou
c) un hybridome.

**3.** Procédé de la revendication 1 dans lequel les gènes apoptotic$^R$ comprennent en outre XIAP$\Delta$.

**4.** Procédé de la revendication 2a) dans lequel la lignée cellulaire CHO consomme le lactate accumulé pendant la phase exponentielle de culture de cellules.

**5.** Procédé de la revendication 2a) dans lequel la lignée cellulaire CHO sécrète moins de lactate pendant la phase exponentielle de culture de cellules qu'une lignée cellulaire CHO ne contenant pas un ou plusieurs gènes apoptotic$^R$.

**6.** Procédé de la revendication 1 dans lequel la densité de cellules viables (VCD) maximale est augmentée.

**7.** Procédé de la revendication 1 dans lequel la longévité de la culture de cellules est prolongée.

**8.** Procédé de la revendication 1 dans lequel le titre de la culture de cellules est augmenté.

**9.** Procédé de la revendication 1 dans lequel le nombre de cellules viables intégré (IVCC) de la culture de cellules est augmenté.

**10.** Procédé de la revendication 1 dans lequel le flux cellulaire de calcium est réduit.

**11.** Procédé de la revendication 1 dans lequel le potentiel membranaire mitochondrial est augmenté.

**12.** Procédé de la revendication 1 dans lequel les gènes d'intérêt codent pour une chaîne lourde d'anticorps et une chaîne légère d'anticorps.

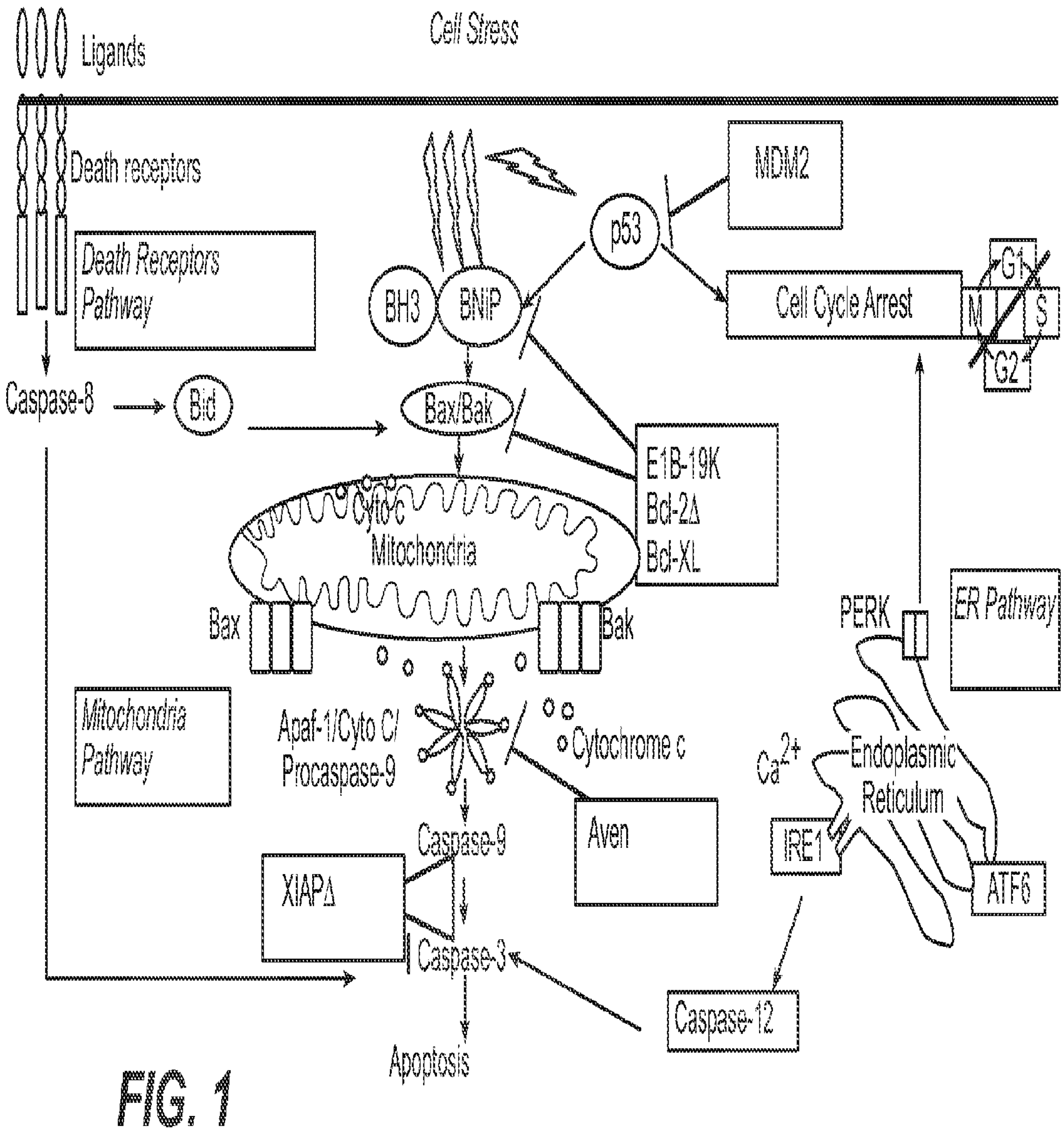
Ligands
Cell Stress
Death receptors
MDM2
Death Receptors Pathway
p53
BH3
BNIP
Cell Cycle Arrest
G1
M
S
G2
Caspase-8
Bid
Bax/Bak
E1B-19K
Bcl-2Δ
Bcl-XL
Cyto c
Mitochondria
Bax
Bak
PERK
ER Pathway
Mitochondria Pathway
Apaf-1/Cyto C/
Procaspase-9
Cytochrome c
Ca2+
Endoplasmic
Reticulum
Aven
Caspase-9
IRE1
XIAPΔ
ATF6
Caspase-3
Caspase-12
Apoptosis

FIG. 1

FIG. 2A

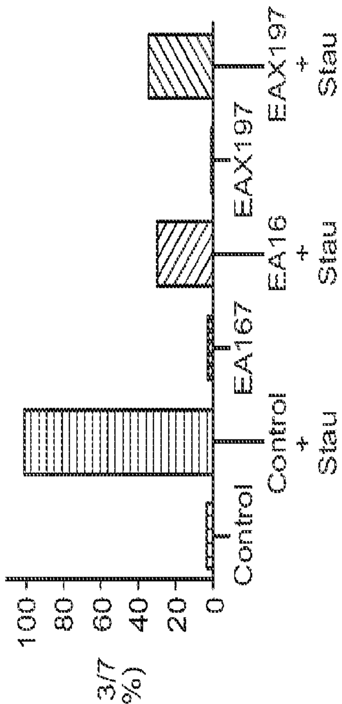


FIG. 2B

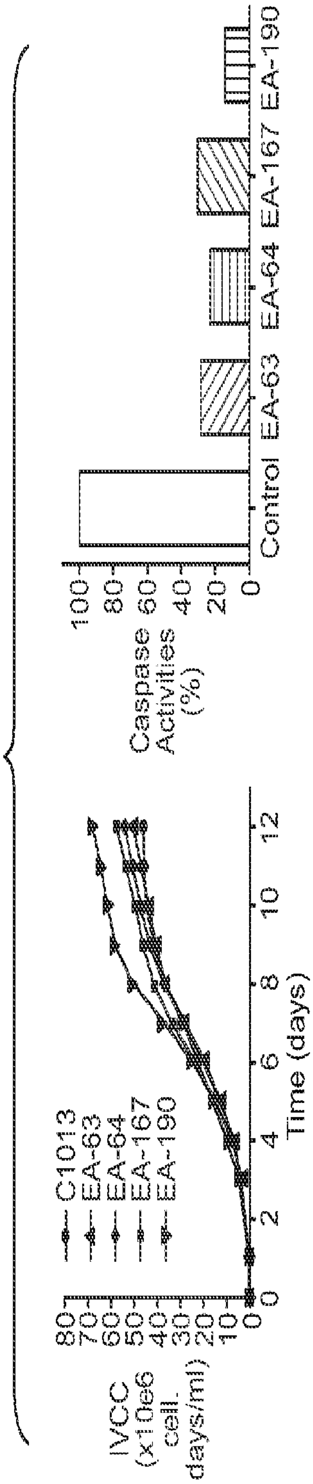


FIG. 2C

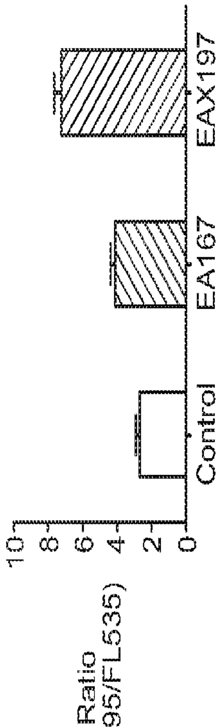

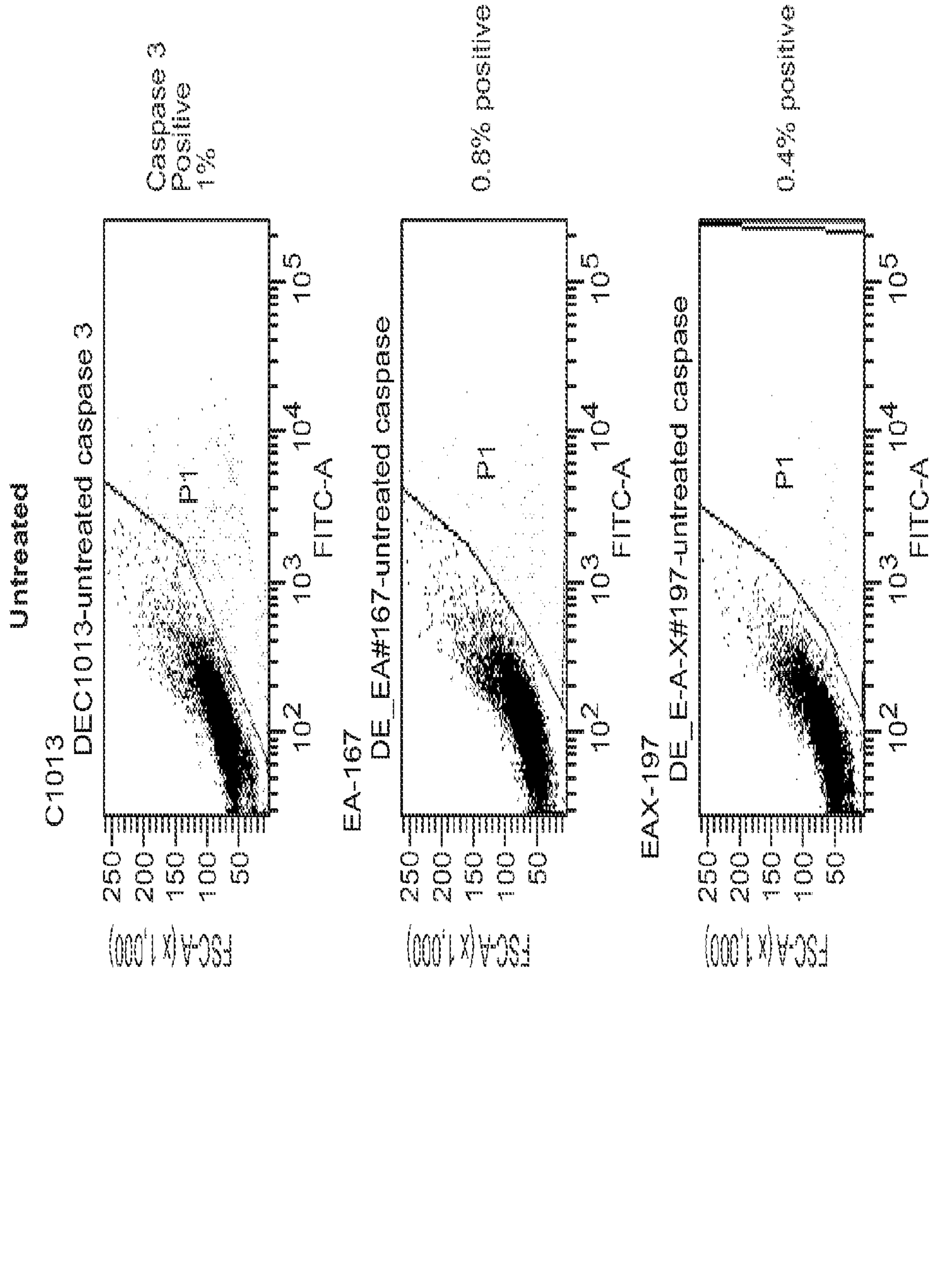
Untreated
C1013
DEC1013-untreated caspase 3
Caspase 3
Positive
1%
P1
FSC-A (x 1,000)
250
200
150
100
50
$10^2$
$10^3$
$10^4$
$10^5$
FITC-A
EA-167
DE_EA#167-untreated caspase
0.8% positive
EAX-197
DE_E-A-X#197-untreated caspase
0.4% positive

FIG. 3A

FIG. 3B

DMSO (Vehicle) treated

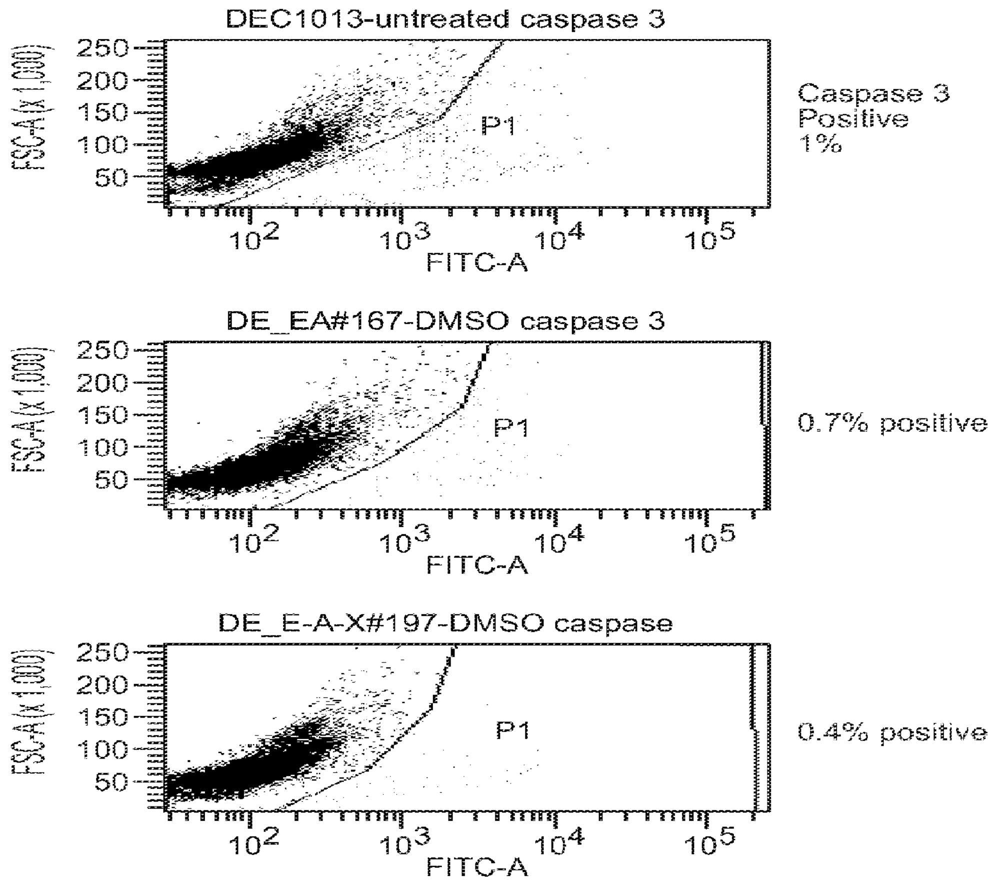

FIG. 3C

Staurosporine treated

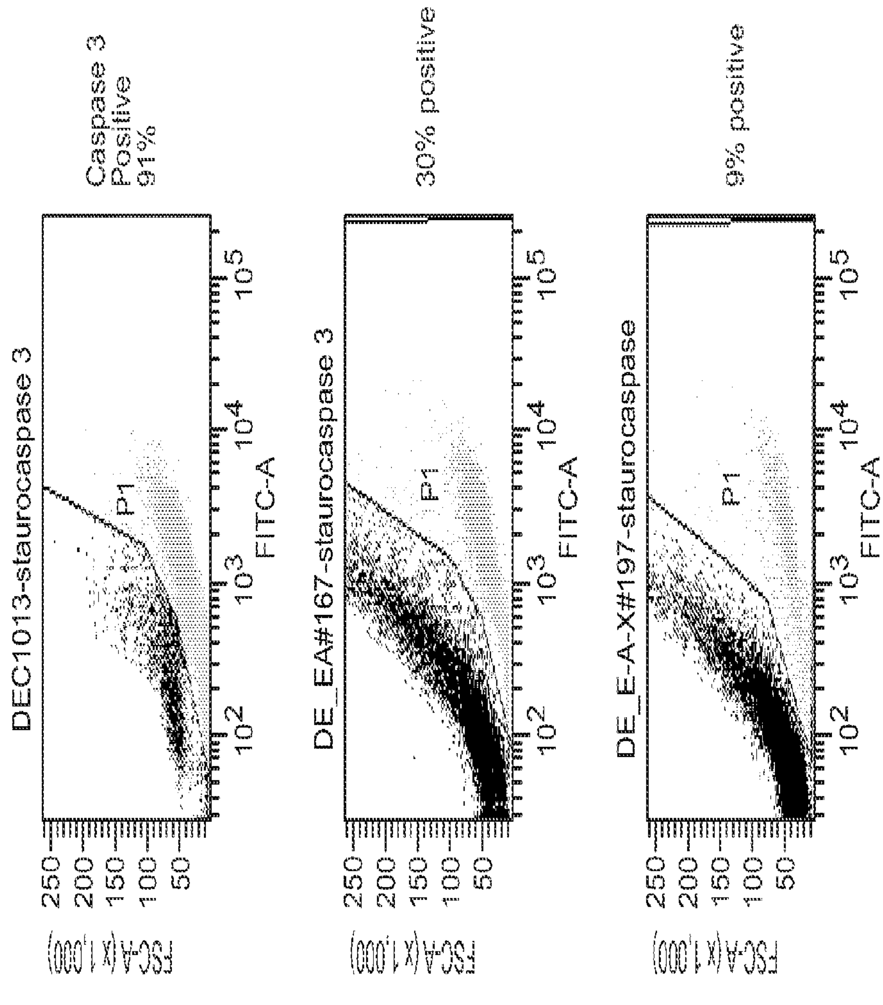

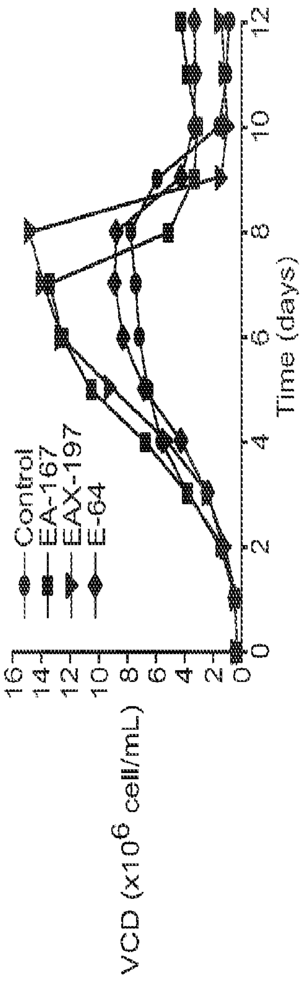


FIG. 4A

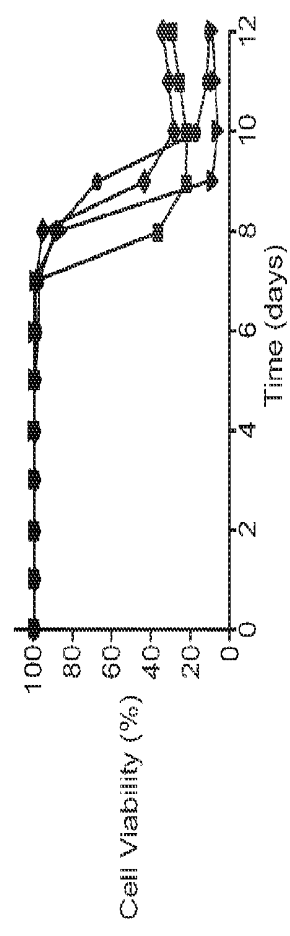


FIG. 4B

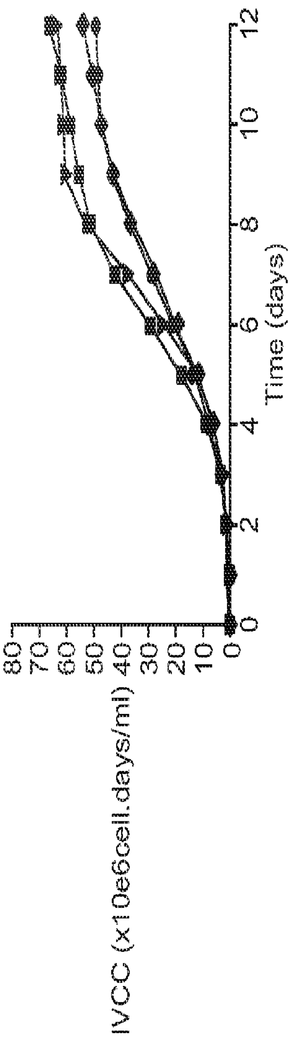


FIG. 4C

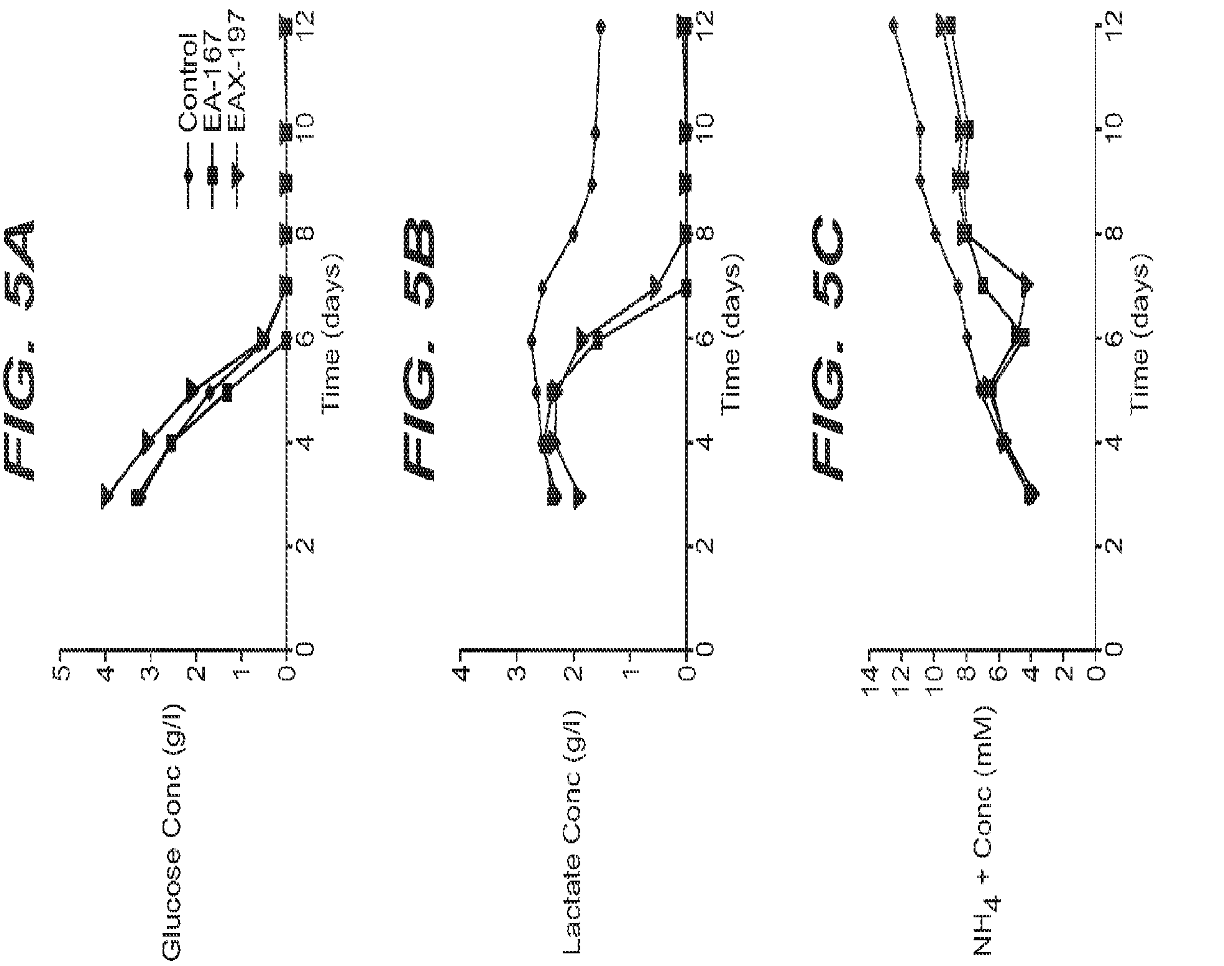


FIG. 5A

FIG. 5B

FIG. 5C

FIG. 6A

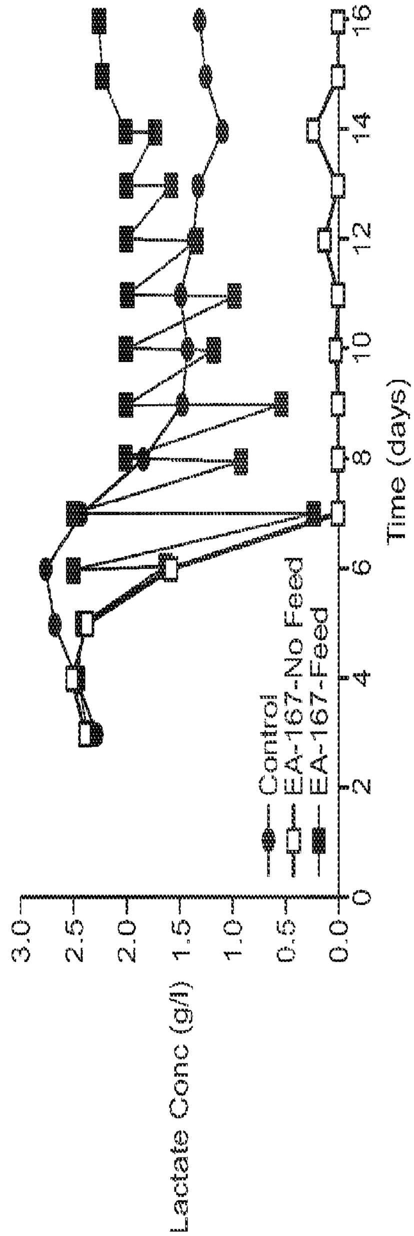
Lactate Conc (g/l)
3.0
2.5
2.0
1.5
1.0
0.5
0.0
0
2
4
6
8
10
12
14
16
Time (days)
Control
EA-167-No Feed
EA-167-Feed

FIG. 6B

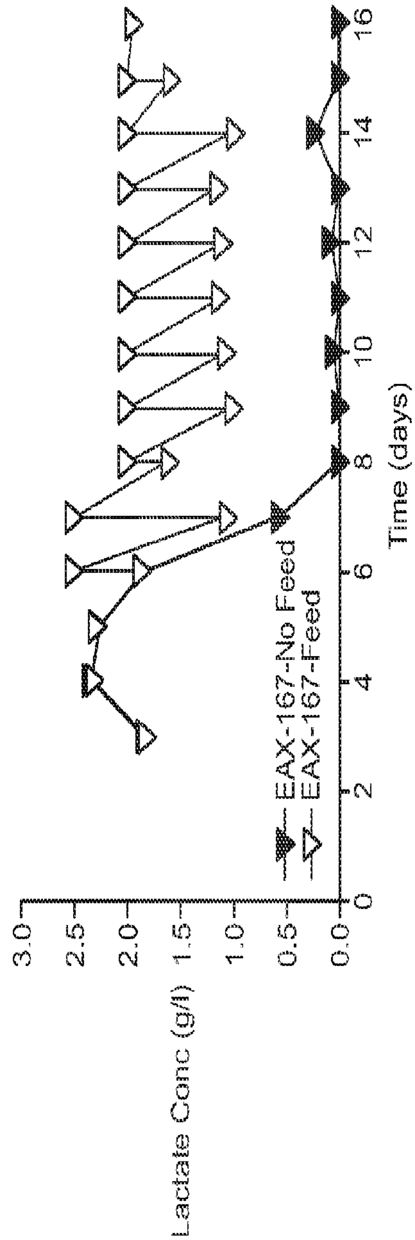
Lactate Conc (g/l)
3.0
2.5
2.0
1.5
1.0
0.5
0.0
0
2
4
6
8
10
12
14
16
Time (days)
EAX-167-No Feed
EAX-167-Feed

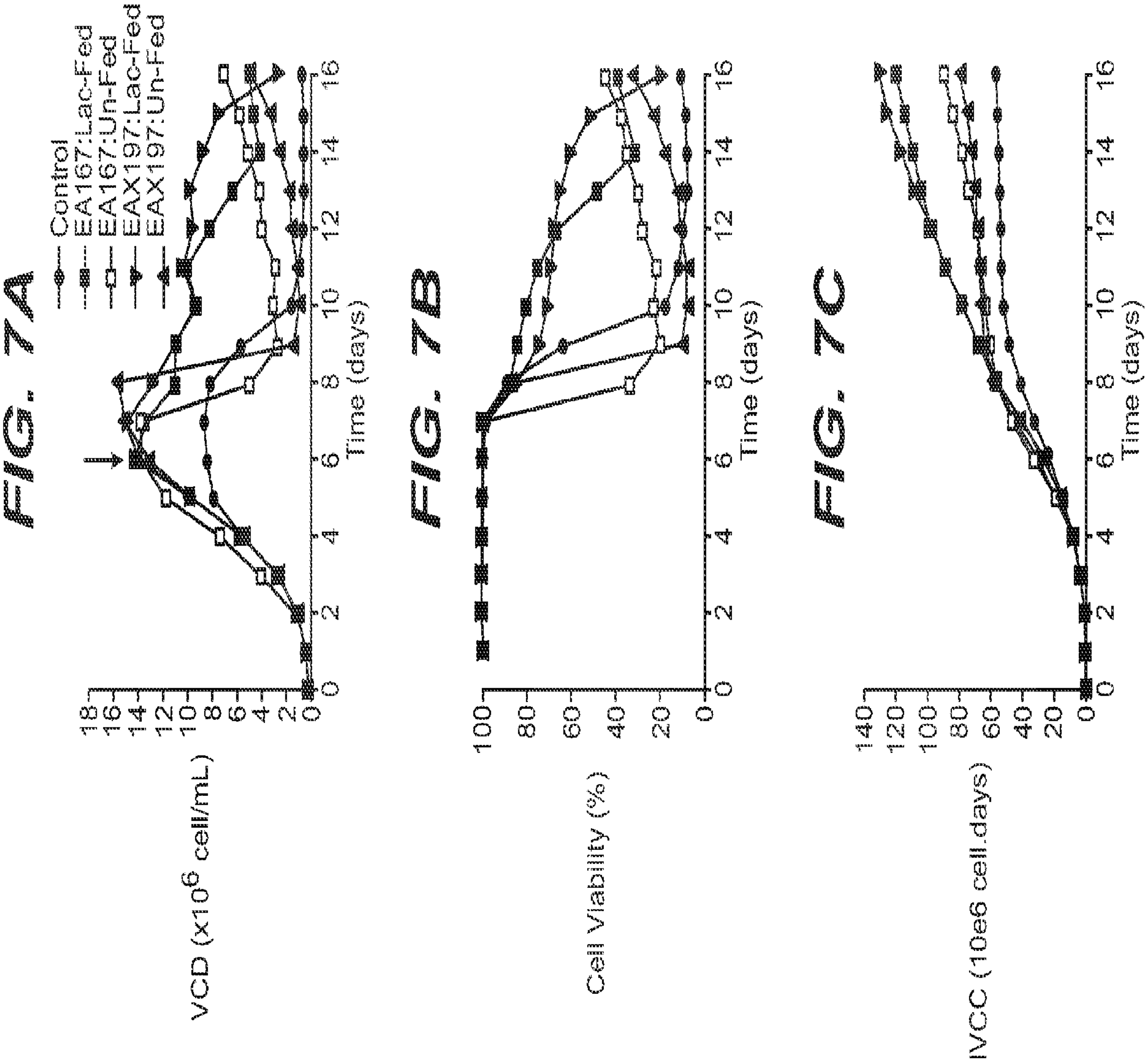


FIG. 7A

FIG. 7B

FIG. 7C

FIG. 7D

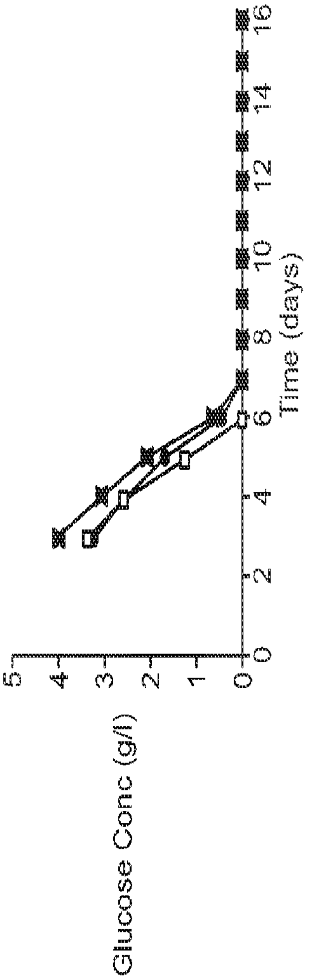


FIG. 7E

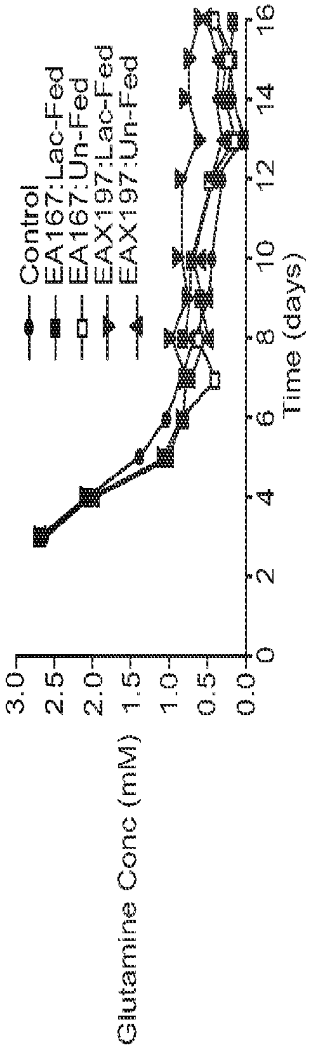


FIG. 7F

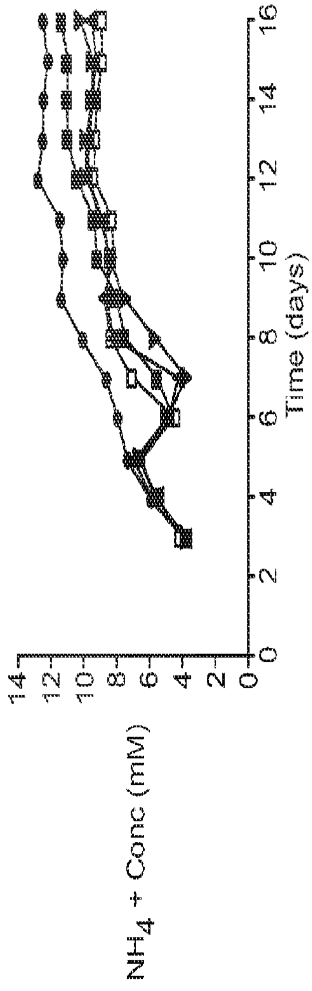

FIG. 7G

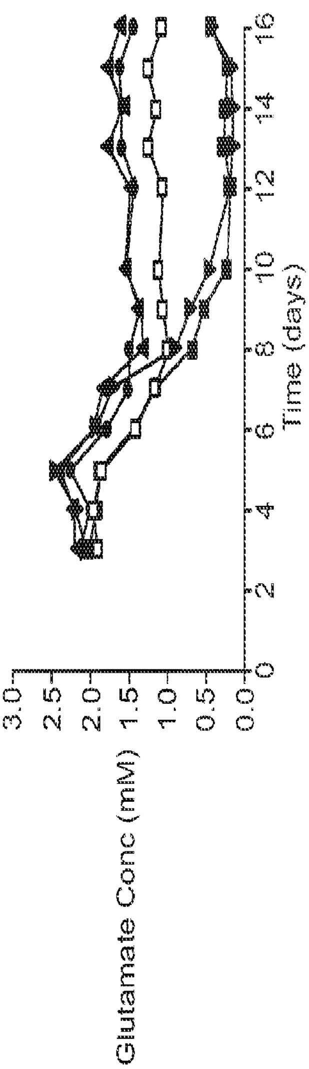
Glutamate Conc (mM)
3.0
2.5
2.0
1.5
1.0
0.5
0.0
0
2
4
6
8
10
12
14
16
Time (days)

FIG. 8A

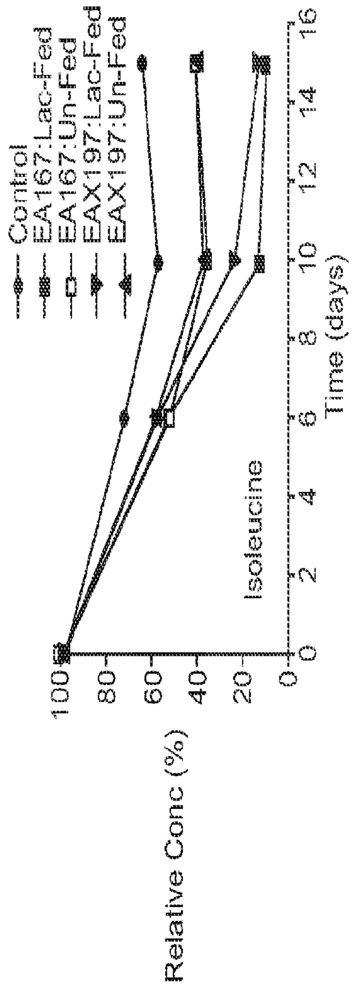


FIG. 8B

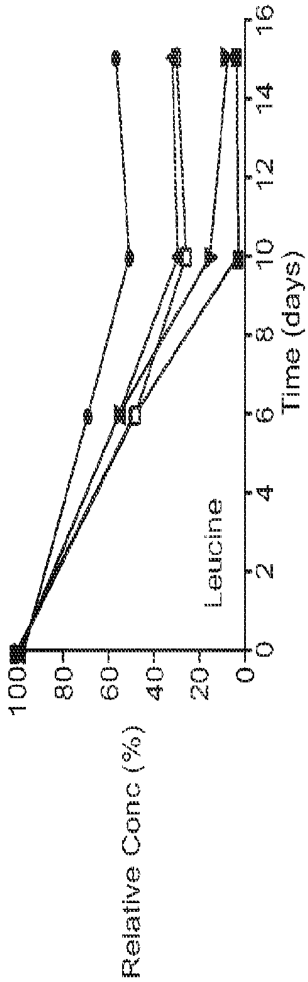


FIG. 8C

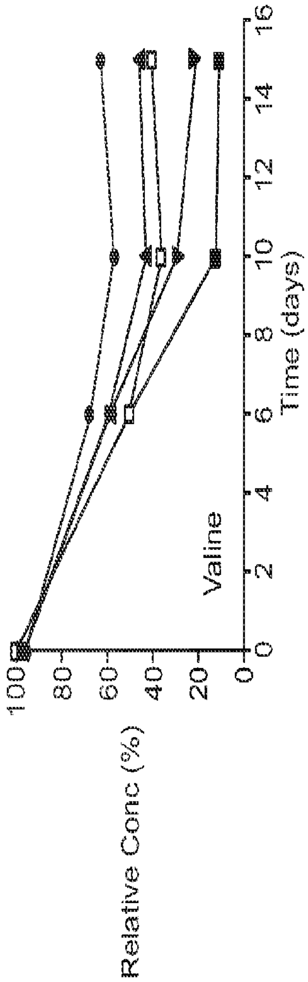

FIG. 8D

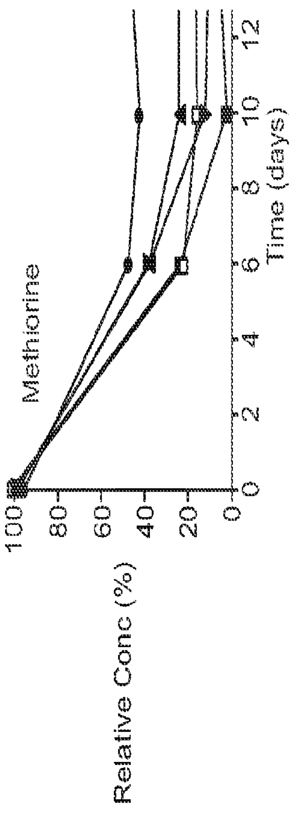


FIG. 8E

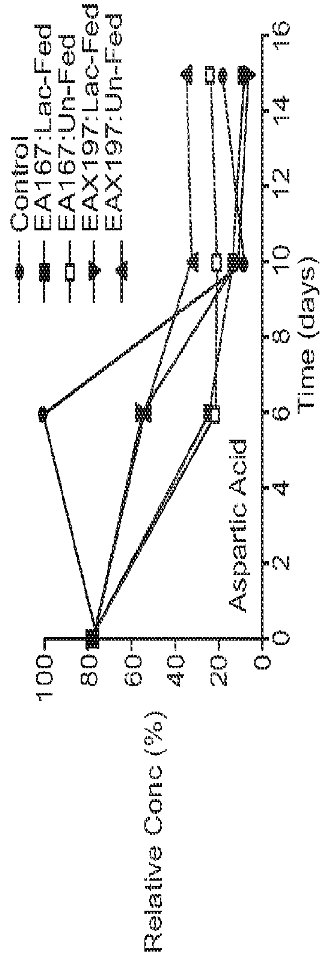


FIG. 8F

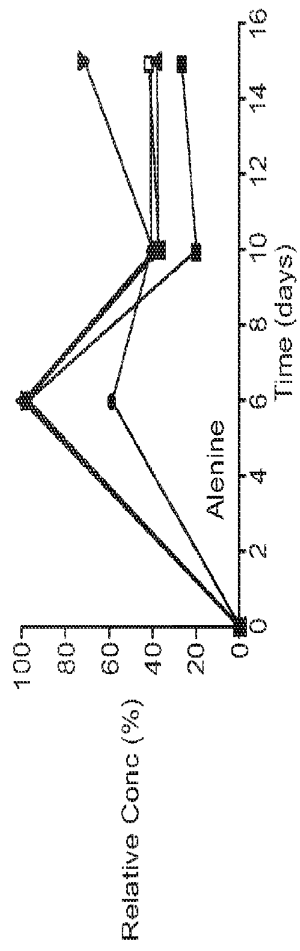

FIG. 8G

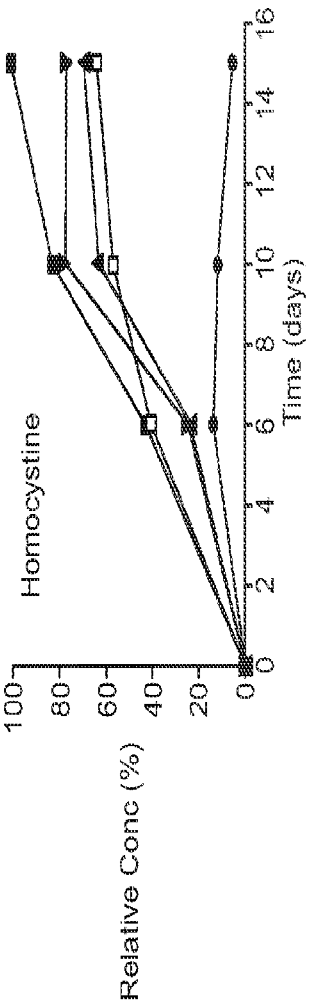

Homocystine
Relative Conc (%)
100
80
60
40
20
0
0
2
4
6
8
10
12
14
16
Time (days)

FIG. 9A

VCD

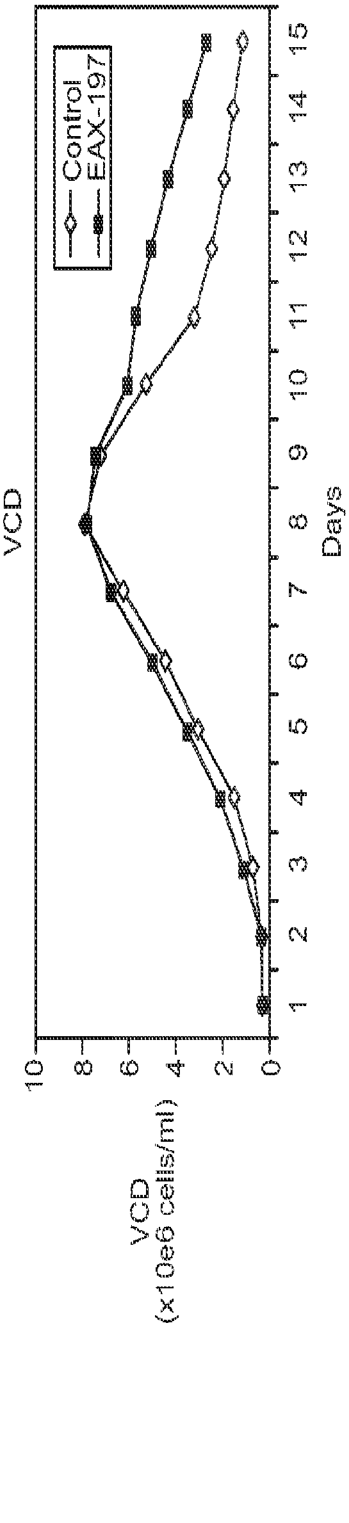


FIG. 9B

Viability

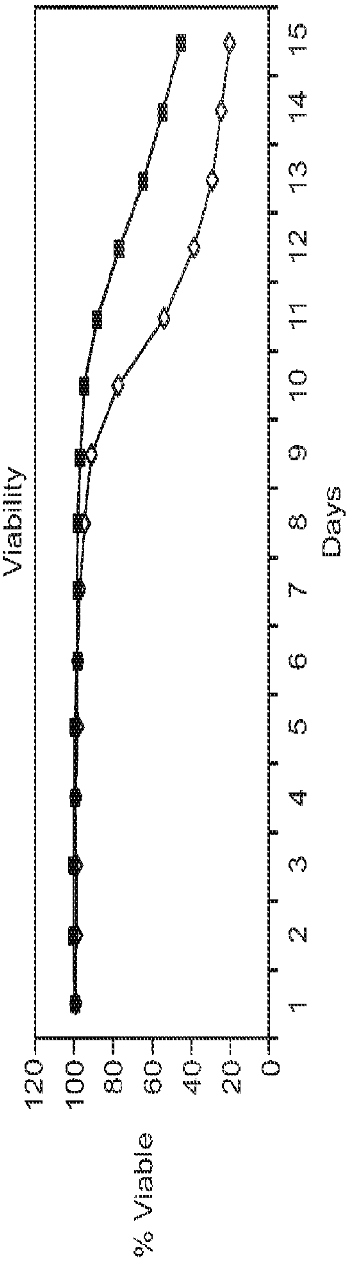

FIG. 9C

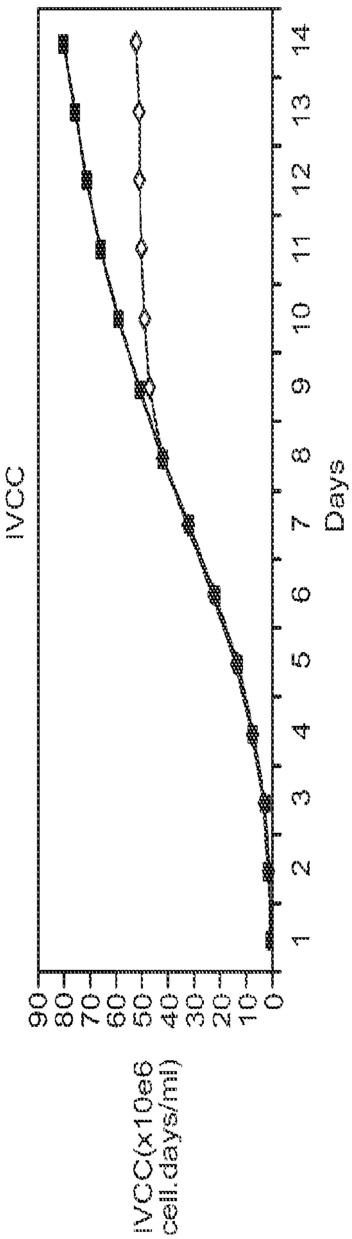
IVCC
IVCC(x10e6 cell.days/ml)
0
10
20
30
40
50
60
70
80
90
Days
1
2
3
4
5
6
7
8
9
10
11
12
13
14

FIG. 9D

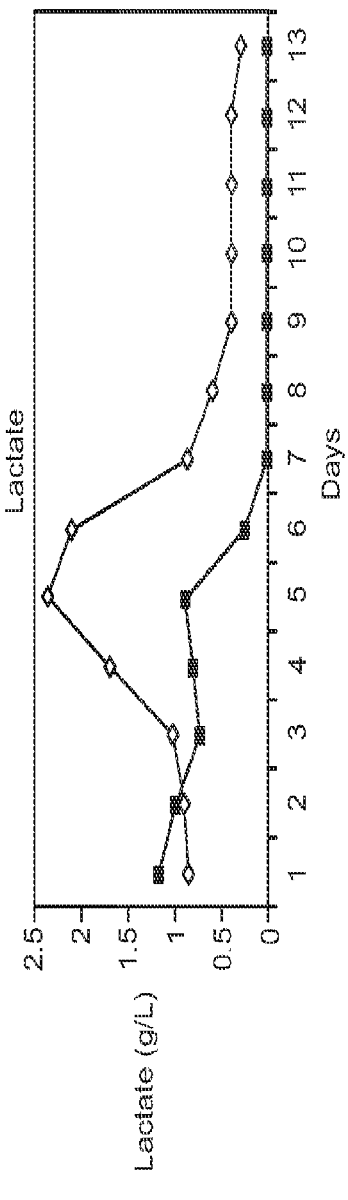
Lactate
Lactate (g/L)
0
0.5
1
1.5
2
2.5
Days
1
2
3
4
5
6
7
8
9
10
11
12
13

FIG. 10A

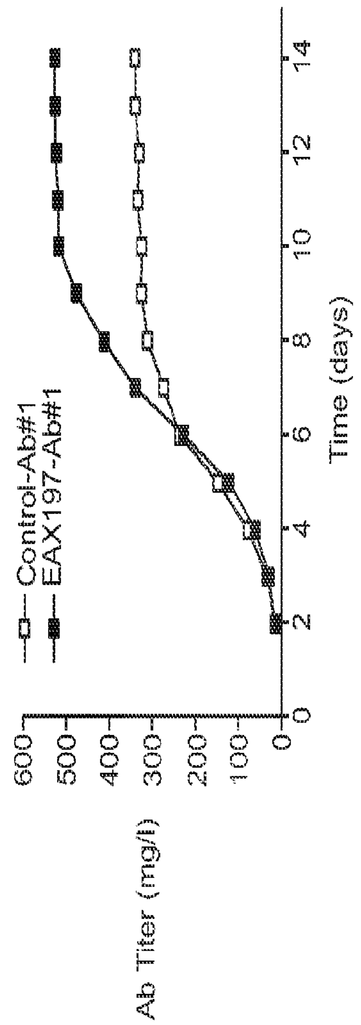


FIG. 10B

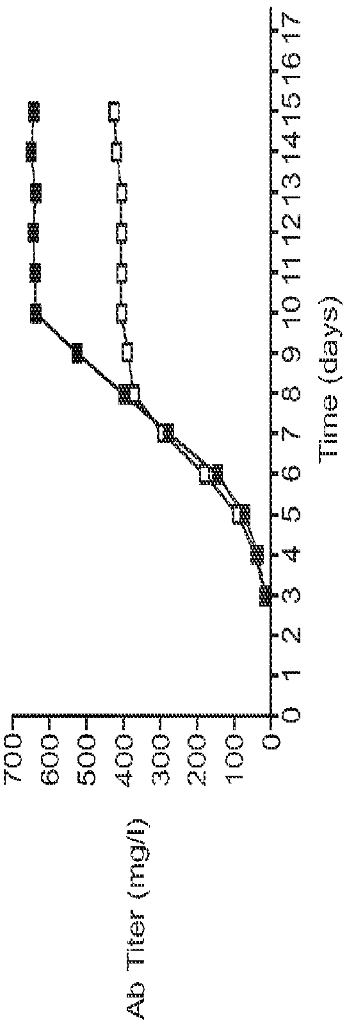

FIG. 11A

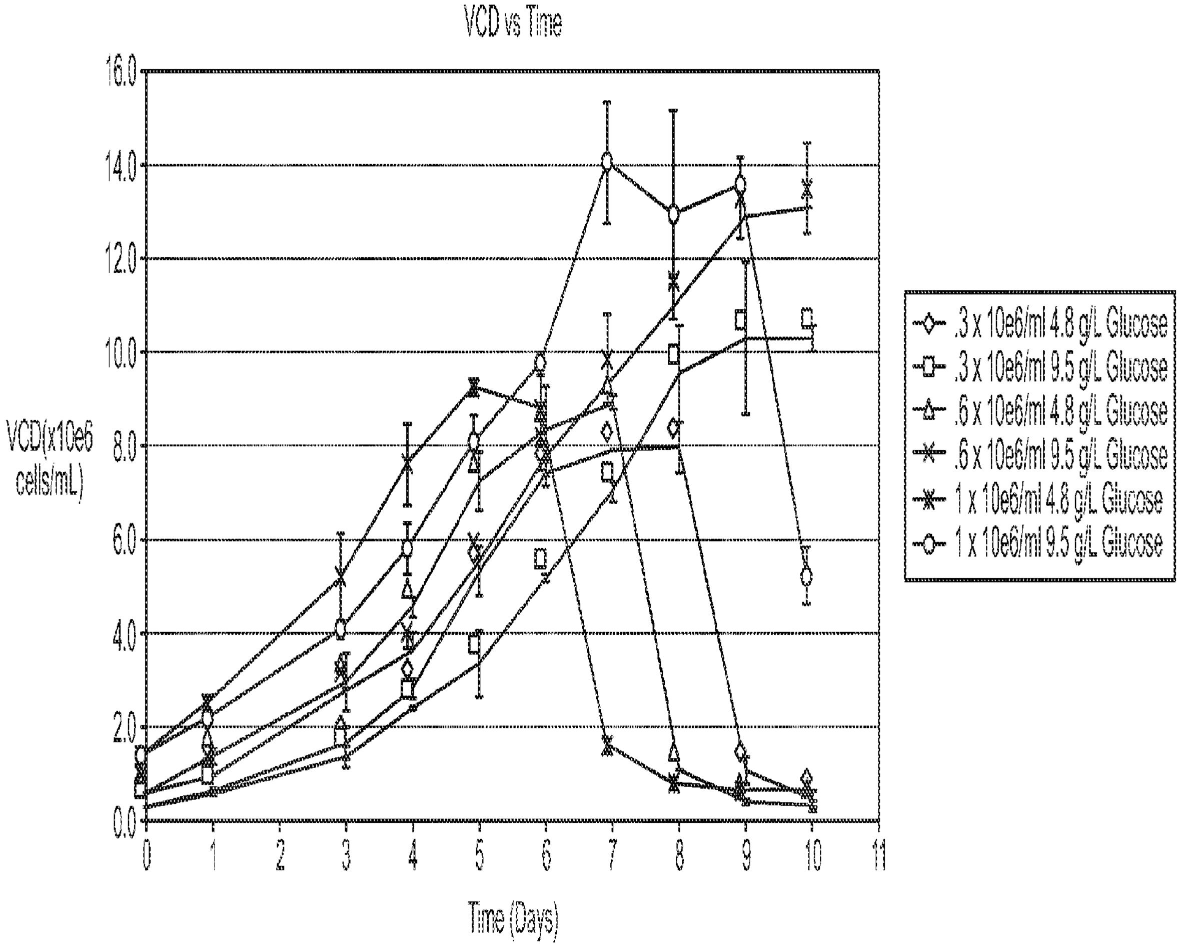
VCD vs Time
16.0
14.0
12.0
10.0
8.0
6.0
4.0
2.0
0.0
VCD(x10e6 cells/mL)
0
1
2
3
4
5
6
7
8
9
10
11
Time (Days)
.3 x 10e6/ml 4.8 g/L Glucose
.3 x 10e6/ml 9.5 g/L Glucose
.6 x 10e6/ml 4.8 g/L Glucose
.6 x 10e6/ml 9.5 g/L Glucose
1 x 10e6/ml 4.8 g/L Glucose
1 x 10e6/ml 9.5 g/L Glucose

FIG. 11B

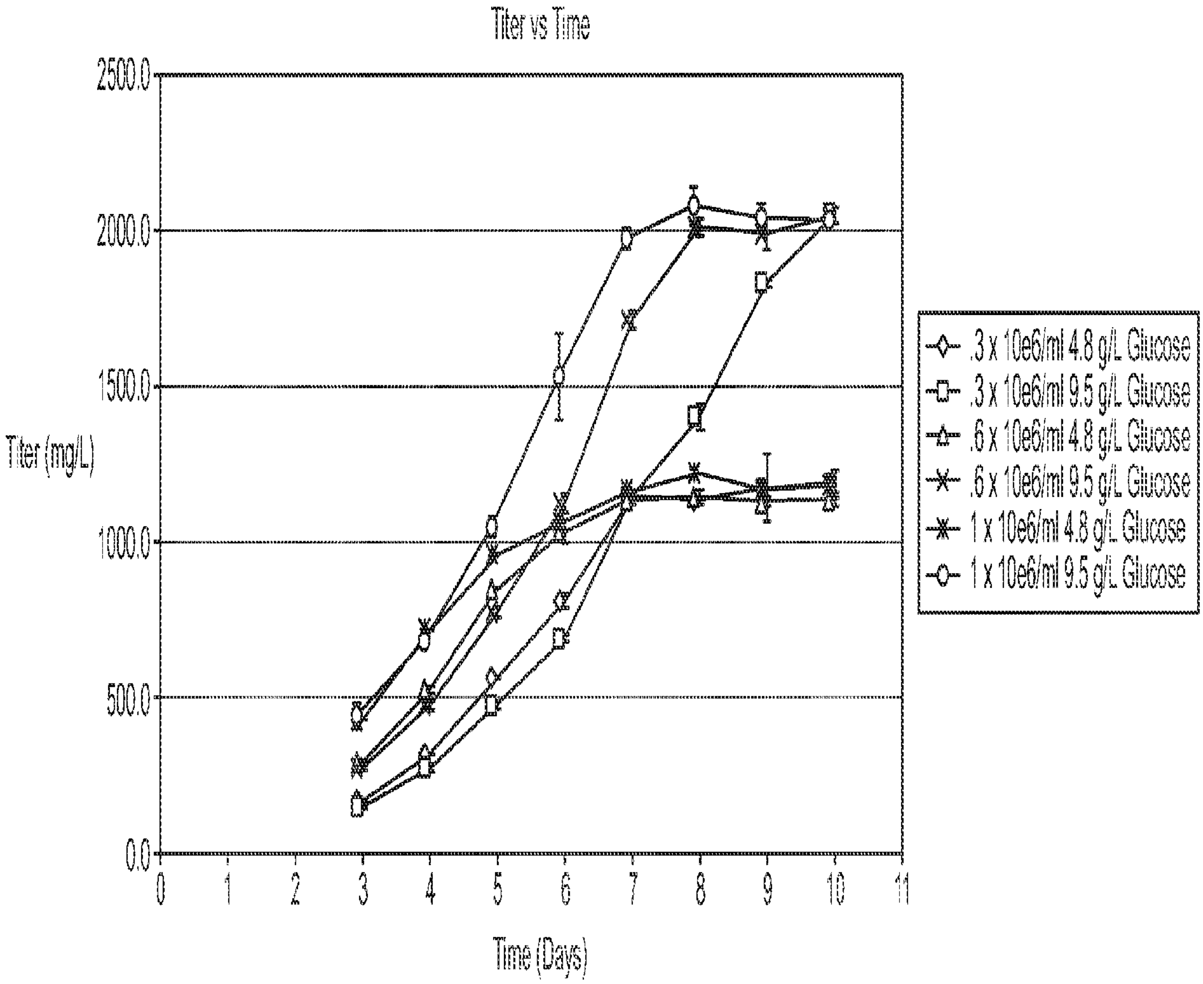
Titer vs Time
2500.0
2000.0
1500.0
1000.0
500.0
0.0
Titer (mg/L)
0
1
2
3
4
5
6
7
8
9
10
11
Time (Days)
.3 x 10e6/ml 4.8 g/L Glucose
.3 x 10e6/ml 9.5 g/L Glucose
.6 x 10e6/ml 4.8 g/L Glucose
.6 x 10e6/ml 9.5 g/L Glucose
1 x 10e6/ml 4.8 g/L Glucose
1 x 10e6/ml 9.5 g/L Glucose

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090042253 A1
- US 20070092947 A
- US 20080015164 A
- US 5672502 A
- US 20050089993 A
- WO 61061233 A


### Non-patent literature cited in the description


- **GOSWAMI et al.** *Biotechnol Bioeng,* 1999, vol. 62, 632-640
- **CHIANG ; SISK.** *Biotechnol Bioeng,* 2005, vol. 91, 779-792
- **FIGUEROA et al.** *Biotechnol Bioeng,* 2001, vol. 73, 211-222
- **METAB.** *Eng,* 2003, vol. 5, 230-245
- *Biotechnol Bioeng,* 2004, vol. 85, 589-600
- **MERCILLE ; MASSIE.** *Biotechnol Bioeng,* 1994, vol. 44, 1140-1154
- **ARDEN ; BETENBAUGH.** *Trends Biotechnol,* 2004, vol. 22, 174-180
- **VIVES et al.** *Metab Eng,* 2003, vol. 5, 124-132
- **BURTEAU et al.** *In Vitro Cell Dev Biol Anim,* 2003, vol. 39, 291-296
- **ZHANG ; ROBINSON.** *Cytotechnology,* 2005, vol. 48, 59-74
- **SAUERWALD et al.** *Biotechnol Bioeng,* 2002, vol. 77, 704-716
- *Biotechnol Bioeng,* 2003, vol. 81, 329-340
- **MASTRANGELO et al.** *Trends Biotechnol,* 1998, vol. 16, 88-95
- **MEENTS et al.** *Biotechnol Bioeng,* 2002, vol. 80, 706-716
- **TEY et al.** *J Biotechnol,* vol. 79, 147-159
- *Biotechnol Bioeng,* 2000, vol. 68, 31-43
- **KUROKAWA et al.** *BiotechnolBioeng,* 1994, vol. 44, 95-103
- **XIE ; WANG.** *Biotechnol Bioeng,* 1993, vol. 43, 1175-1189
- **ZHANG et al.** *J Chem Technol Biotechnol,* 2004, vol. 79, 171-181
- **ZHOU et al.** *Biotechnol Bioeng,* vol. 46, 579-587
- **MARTINELLE et al.** *Biotechnol Bioeng,* 1998, vol. 60, 508-517
- **ALTAMIRANO et al.** *J Biotechnol,* 2004, vol. 110, 171-179
- *J Biotechnol,* 2006, vol. 125, 547-556
- **WALSCHIN ; WU.** *J Biotechnol,* 2007, vol. 131, 168-176
- **MAJORS et al.** *Metab Eng,* 2007, vol. 9, 317-326
- **WHITE et al.** *Nat Cell Biol,* 2005, vol. 7, 1021-1028
- **FIGUEROA et al.** *Metab. Eng.,* vol. 5, 230-245
- **NIVITCHANYONG et al.** *Biotechnol Bioeng,* 2007, vol. 98, 825-841
- **SAUERWALD et al.** *Biotechnol Bioeng,* 2006, vol. 94, 362-369
- **FIGUEROA et al.** *Biotechnol Bioeng,* 2007, vol. 97 (4), 877-892
- **CORY et al.** *Oncogene,* 2003, vol. 22, 8590-8607
- **YANG et al.** *Science,* 1997, vol. 275, 1129-1132